# EUROPEAN PATENT SPECIFICATION

(11) **EP 1 796 751 B1**
(45) Date of publication and mention of the grant of the patent: **04.05.2011**
(21) Application number: 05782881.6
(22) Date of filing: 12.09.2005
(51) Int. Cl.: A61L 27/60

(54) **TRANSGLUTAMINASE CROSSLINKED COLLAGEN BIOMATERIAL FOR MEDICAL IMPLANT MATERIALS**
TRANSGLUTAMINASE-VERNETZTES KOLLAGENBIOMATERIAL FÜR MEDIZINISCHE IMPLANTATIONSMATERIALIEN
BIOMATERIAU EN COLLAGENE RETICULE AVEC DE LA TRANSGLUTAMINASE POUR MATERIAUX POUR PROTHESE MEDICALE.

(30) Priority: 10.09.2004 GB 0420091
(43) Date of publication of application: 20.06.2007
(73) Proprietor: Aston University, Birmingham B4 7ET (GB)
(72) Inventor: GRIFFIN, Martin, Bleasby, Nottinghamshire NG14 7GH (GB); COLLIGHAN, Russell, Birmingham B4 7ET (GB); CHAU, David, Birmingham B4 7ET (GB); VERDERIO EDWARDS, Elisabetta, Nottingham NG11 8NS (GB)
(74) Representative: Smith, Stephen Edward
(86) International application number: PCT/GB2005/003520
(87) International publication number: WO 2006/027622

(56) References cited:
- WO-A-97/40701
- ERWANTO Y ET AL: "EFFECT OF HEATING ON POLYMERIZATION OF PIG SKIN COLLAGEN USING MICROBIAL TRANSGLUTAMINASE" ASIAN-AUSTRALASIAN JOURNAL OF ANIMAL SCIENCES, SUWEON, KR, vol. 15, no. 8, 2002, pages 1204-1209, XP001182377 ISSN: 1011-2367
- ORBAN JANINE M ET AL: "Crosslinking of collagen gels by transglutaminase" J. BIOMED. MATER. RES. PART A; JOURNAL OF BIOMEDICAL MATERIALS RESEARCH - PART A MAR 15 2004, vol. 68, no. 4, 15 March 2004 (2004-03-15), pages 756-762, XP002391635

## Description

The present invention relates to material for use in medicine, in particular medical implant materials. Specifically, the invention provides a method of improving the biocompatibility of a medical implant material.

### Background

The shortage of organ or tissue donors has required the use of new biological substitutes regenerated from tissue cells or synthetic polymer matrices. From which, tissue, replacement has become an important part of modem medical treatments, whether artificial, such as joint replacements or living, such as skin and organ transplants. A new alternative for the medical industry is the use of artificial living tissues designed to mimic the native tissue and induce tissue formation. Replacement of skin with artificial collagen-GAG matrices has been investigated since the early 1980s and is now in clinical use (Bell *et al.,* 1981; Burke *et al.,* 1981). Tissue engineering materials must satisfy several crucial factors: they must be resorbable, they must not elicit inflammation or a foreign body response, they must possess adequate mechanical strength to perform its on-site function and they must encourage and promote cellular invasion, proliferation and differentiation. At its simplest characteristic, the material serves as a bridge guiding cell-mediated remodelling to reproduce the structure and organisation of the intended tissues.

Although many matrices currently exist and have been optimised for their individual applications; not many materials have general multi-application capabilities. Synthetic biodegradable polymers, such as aliphatic polyester, (*e.g*. polyglycolic acid, polylactic acid, polyesters and their copolymers, are the most commonly used for tissue engineering applications. However, these synthetic polymers posses a surface chemistry that does not promote general cell adhesion. In addition, they can produce high local concentrations of acidic by-products during degradation that may induce adverse inflammatory responses or create local environments that may not favour the biological activity of surrounding cells (Sachlos *et al.,* 2003). Hydrogels have gained popularity as potential materials for tissue engineering due to their high water content, good biocompatibility, and consistency similar to soft tissue. (Schmedlen *et al.,* 2002). However, because of their complex, three-dimensional hydrophobic structure, they are capable of absorbing excess amounts of aqueous solution and undergoing degradation via erosion, hydrolysis, solubilisation and other biodegradation mechanisms. (Einerson *et al.,* 2002). Other bioactive materials, such as glasses, ceramics or gels, possess unsuitable physical and mechanical characteristics that prevent them from being used in many applications. Additionally, many of these have not had their biological activity assessed using in vitro cell culture systems. (Rhee *et al.,* 2003).

Collagen is the major component of skin bones and connective tissue. Collagen is a very popular biomaterial due to its biocompatibility; the ability to support cell adhesion and proliferation. It is also biodegradable and only weakly antigenic, and is thus able to persist in the body without developing a foreign body response that could lead to its premature rejection (Goo *et al.,* 2003). Nevertheless, the primary reason for the usefulness of collagen in biomedical application is that collagen can form fibres with extra strength and stability through its self-aggregation and cross-linking (Lee *et al.;* 2001). Unfortunately, collagen, like many natural polymers once extracted from its original source and then reprocessed, suffers from weak mecliauical properties, thermal instability and ease of proteolytic breakdown. To overcome these problems, collagen has been cross-linked by a variety of agents and is the subject of much recent research to find methods of preventing rapid absorption by the body. This has been accomplished by the use of cross-linking agents such as glularaldehyde (Barbani *et al.,* 1995), formaldehyde (Ruderman *et al*., 1973), chrome tanning (Bradley and Wilkes, 1977), epoxy compounds (To *et al.,* 1993), acyl azide (Petite *et al.,* 1990), carbodiimides (Nimni *et al*., 1993) and hexamethylenediisocyanate (Chvapil *et al*., 1993). The use of UV light, gamma irradiation and dehyrothermal treatment has also shown to be effective at introducing cross-links into collagen (Harkness *et al.,* 1966; Stenzel *et al,* 1969; Miyata *et al.,*1971; Gorham *et al*., 1992). However, these methods suffer from the problem that the residual catalysts, initiators and unreacted or partially reacted cross-linking agents used can be toxic or cause inflammatory responses if not fully removed or, simply, not cost-effective or practical at the large-scale (Matsuda *et al.,* 1999; Ben-Slimane *et al*., 1988; Dunn *et al*., 1969).

WO 97/90701 describes transglutaminase cross-linked protein gels having improved strength and thermal stability, which may optionally contain mon-proteinaceous compounds having attachment sites for cells. However, this patent application does not provide any enzyme-based methods for improving the biocompatibility of medical implant materials

Hence, the present invention seeks to provide improved biomaterials which overcome the above problems of existing biomaterials.

### Summary of the Invention

A first aspect of the invention provides a use of a transglutaminase for improving the biocompatibility of a biomaterial comprising collagen by crosslinking the collagen. Thus, the method comprises treating collagen with a transglutaminase under conditions which permit the formation of crosslinks within the collagen,

By 'biomaterial' we include any material comprising collagen which is suitable for use within or on a mammalian host body (and, in particular, a human host body). Preferably, the biomaterial is suitable for use as a medical implant material and/or a wound dressing.

By 'biocompatible' we mean the biomaterial is able to support its colonisation by host cells and their proliferation therein. Thus, biocompatibility is not intended to cover mere adhesion of host cells to the biomaterial, but rather relates to an interaction between the host cells and biomaterial which permits colonisation to occur. Specifically, biocompatibility includes the ability of said material to support cell attachment, cell spreading, cell proliferation and differentiation.

Thus, the biocompatible biomaterial exhibits an enhanced ability to support cell attachment, cell spreading, cell proliferation and differentiation compared to non-crosslinked collagen.

Advantageously, the biomaterial exhibits an enhanced ability to support attachment, spreading, proliferation and/or differentiation of osteoblasts compared to non-crosslinked collagen.

Thus, the invention relates to a method of improving the biocompatibility of collagen. Biocompatibility of a biomaterial such as collagen may be assessed using methods known in the art (see Examples). For example, increased biocompatibility of a biomaterial is associated witch an increase in the ability of the material to facilitate cell attachment, cell spreading, cell proliferation and differentiation. In addition, the biomaterial should not induce any substantial loss in cell viability, *i.e.* via the induction of cell death through either apoptosis or necrosis. The differentiation of a cell type is measured in different ways depending on the cell type in question. For example, for osteoblasts cells in culture, allcaline phosphate together with extracellular matrix (ECM) deposition, *e.g*. collagen 1, fibronectin, osteonectin and osteopontin, can be used as a marker. In addition, the ability of cells to proliferate and deposit ECM is important to any material that is to be used as an implant, this includes endothelial cells, chondrocytes and epithelial cells *etc.*

In a further preferred embodiment of the first aspect of the invention, the biocompatible biomaterial exhibits enhanced resistance to cell-mediated degradation compared to non-crosslinked collagen. In particular, the biocompatible biomaterial preferably exhibits enhanced resistance to one or more protease enzymes produced by mammalian cells.

It will be appreciated by persons skilled in the art that the invention may be used to improve the biocompatibility of any collagen-based starting material, provided that the collagen is present in sufficient concentration to enable successful formation of a solid gel matrix. Preferably, the collagen-based starting material comprises collagen at a concentration of 1 to 10 mg/ml.

Preferably, the collagen-containing starting material consists of substantially pure collagen. By 'substantially pure' we mean that the starting material is at least 50% by weight collagen, preferably at least 60%, 70%, 80%, 90%, or 95% by weight collagen. More preferably, the starting material is 100% by weight collagen.

Alternatively, the collagen-containing starting material may comprise one or more additives. For example, in a preferred embodiment the starting material comprises a cell adhesion factor.

By 'cell adhesion factor' we mean a component (*e.g*. polypeptide) that possesses specific binding sites for cell surface receptors, thus enabling cell attachment, cell spreading and differentiation.

Preferably, the cell adhesion factor is selected from the group consisting of fibronectin, fibrin, fibrillin, glycosoaminoglycans, hyaluronic acid laminin, vitronectin and elastin.

More preferably, the cell adhesion factor is fibronectin.

Most preferably, the fibronectin is present at a concentration of 5 to 1000 µg/ml.

In a further preferred embodiment, the additives is selected from the group consisting of polylactic acid, polyhydroxybutyrate, poly(ε-caprolactone), polyglycolic acid, polysaccharides, chitosans and silicates.

In a further preferred embodiment, the collagen-containing biomaterial could is coated on an inert medical implant, such as metals, bioceramics, glass or bio-stable polymers (for example polyethylene, polypropylene, polyurethane, polytetrafluoroethylene, poly(vinyl chloride), polyamides, poly(methylmethacrylate), polyacetal, polycarbonate, poly(-ethylene terphthalate), polyetheretherketone, and polysulfone). The biomaterial may also be coated or mixed with silk.

A characterising feature of the present invention is that a transglutaminase enzyme is used as a crosslinking agent in place of existing chemical and physical crosslinking means.

Transglutaminases (Enzyme Commission System of Classification 2.3.2.13) are a group of multifunctional enzymes that cross-link and stabilise proteins in tissues and body fluids (Aeschlimann & Paulsson, 1994 & Greenberg *et al.,* 1991). In mammals, they are calcium dependent and catalyse the post-translational modification of proteins by forming inter and intra-molecular ε(γ-glutamyl)lysine cross-links. The bonds that form are stable, covalent and resistant to proteolysis, thereby increasing the resistance of tissues to chemical, enzymatic and physical disruption. In contrast to transglutaminases of mammalian origin, microbial transglutaminases are generally not Ca²⁺-dependent,

It will be appreciated that the term 'transglutaminase' is intended to include any polypeptide, or derivative thereof which is able to catalyse the formation of inter- and/or intra-molecular ε(γ-glutamyl)lysine crosslinks in collagen. Thus, the transglutaminase may be a naturally occurring transglutaminase, or a variant, fragment of derivative thereof which retains transglutaminase crosslinking activity.

In a preferred embodiment of the first aspect of the invention the transglutaminase is a tissue transglutaminase. Alternatively, a plasma transglutaminase may be used. Preferably, the transglutaminase is derived or prepared from mammalian tissue or cells. For example, the transglutaminase may be guinea pig liver tissue transglutaminase.

More preferably, the transglutaminase is prepared from human tissue or cells. For example, the transglutaminase may be extracted from human tissue sources such as lung, liver, spleen, kidney, heart muscle, skeletal muscle, eye lens, endothelial cells, erythrocytes, smooth muscle cells, bone and macrophages. Advantageously, the transglutaminase is a tissue transglutaminase derived from human red cells (erythrocytes), or a plasma transglutaminase derived from either human placenta or human plasma.

Alternatively, the transglutaminase may be obtained from a culture of human cells that express a mammalian transglutaminase, using cell culture methodology well known in the art. Preferred cell line sources of such transglutaminases include human endothelial cell line ECV304 (for tissue transglutaminase) and human osteosarcoma cell line MG63.

It will be appreciated by those skilled in the art that the source of the transglutaminase may be selected according to the particular use (*e.g*. site of implantation) of the biomaterial. For example, if the biomaterial is to be used as artificial bone, it may be beneficial for the material to comprise a bone-derived transglutaminase.

In an alternative embodiment of the first aspect of the invention, the transglutaminase is a microbial transglutaminase. For example, the transglutaminase may be derived or prepared from *Streptoverticillium mobaraenase, Streptoverticillium ladakanum, Streptoverticillium cinnamoneum, Bacillus subtilis* or *Phytophthora cactorum.*

It will be appreciated by skilled persons that the transglutaminase used in the methods of the invention may be a recombinant transglutaminase.

Nucleic acid molecules encoding a transglutaminase are known in the art. For example, the coding sequence for human coagulation factor XIII A1 polypeptide is disclosed in Grundmann *et al.,* 1986 (accession no. NM 000129). The coding sequence for human tissue transglutaminase is disclosed in Gentile *et al.,* 1991 (accession no. M 55153).

Nucleic acid molecules encoding a transglutaminase may be used in accordance with known techniques, appropriately modified in view of the teachings contained herein, to construct an expression vector, which is then used to transform an appropriate host cell for the expression and production of the polypeptide of the invention. Methods of expressing proteins in recombinant cells lines are widely known in the art (for example, see Sambrook & Russell, 2001, Molecular Cloning, A Laboratory Manual, Third Edition, Cold Spring Harbor, New York). Exemplary techniques also include those disclosed in US Patent Nos. 4,440,859 issued 3 April 1984 to Rutter et al*,* 4,530,901 issued 23 July 1985 to Weissman, 4,582,800 issued 15 April 1986 to Crowl, 4,677,063 issued 30 June 1987 to Mark et al*,* 4,678,751 issued 7 July 1987 to Goeddel, 4,704,362 issued 3 November 1987 to Itakura et al*,* 4,710,463 issued 1 December 1987 to Murray, 4,757,006 issued 12 July 1988 to Toole, Jr. et al*,* 4,766,075 issued 23 August 1988 to Goeddel et al and 4,810,648 issued 7 March 1989 to Stalker;

The nucleic acid molecule, *e.g*. cDNA, encoding the transglutaminase may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA is inserted into an expression vector, such as a plasmid, in proper orientation and correct reading frame for expression. If necessary, the DNA may be linked to the appropriate transcriptional and translational regulatory control nucleotide sequences recognised by the desired host, although such controls are generally available in the expression vector. Thus, the DNA insert may be operatively linked to an appropriate promoter. Bacterial promoters include the *E. coli lacI* and *lacZ* promoters, the T3 and T7 promoters, the *gpt* promoter, the phage λ PR and PL promoters, the *phoA* promoter and the *trp* promoter. Eukaryotic promoters include the CMV immediate early promoter, the HSV thymidine kinase promoter, the early and late SV40 promoters and the promoters of retroviral LTRs. Other suitable promoters will be known to the skilled artisan. Alternatively, the Baculovirus expression system in insect cells may be used (see Richardson *et al.,* 1995). The expression constructs will desirably also contain sites for transcription initiation and termination, and in the transcribed region, a ribosome binding site for translation. (see WO 98/16643)

The vector is then introduced into the host through standard techniques. Generally, not all of the hosts will be transformed by the vector and it will therefore be necessary to select for transformed host cells. One selection technique involves incorporating into the expression vector a DNA sequence marker, with any necessary control elements, that codes for a selectable trait in the transformed cell. These markers include dihydrofolate reductase, G418 or neomycin resistance for eukaryotic cell culture, and tetracyclin, kanamycin or ampicillin resistance genes for culturing in *E.coli* and other bacteria. Alternatively, the gene for such selectable trait can be on another vector, which is used to co-transform the desired host cell.

Host cells that have been transformed by the recombinant DNA of the invention are then cultured for a sufficient time and under appropriate conditions known to those skilled in the art in view of the teachings disclosed herein to permit the expression of the transglutaminase, which can then be recovered.

The recombinant transglutaminase can be recovered and purified from recombinant cell cultures by well-known methods including ammonium sulphate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Many expression systems are known, including systems employing: bacteria (*e.g. E. coli* and *Bacillus subtilis*) transformed with, for example, recombinant bacteriophage, plasmid or cosmid DNA expression vectors; yeasts (*e.g. Saccharomyces cerevisiae*) transformed with, for example, yeast expression vectors; insect cell systems transformed with, for example, viral expression vectors (*e.g*. baculovirus); plant cell systems transfected with, for example viral or bacterial expression vectors; animal cell systems transfected with, for example, adenovirus expression vectors.

The vectors include a prokaryotic replicon, such as the Col E1 *ori,* for propagation in a prokaryote, even if the vector is to be used for expression in other, non-prokaryotic cell types. The vectors can also include an appropriate promoter such as a prokaryotic promoter capable of directing the expression (transcription and translation) of the genes in a bacterial host cell, such as *E. coli,* transformed therewith.

A promoter is an expression control element formed by a DNA sequence that permits binding of RNA polymerase and transcription to occur. Promoter sequences compatible with exemplary bacterial hosts are typically provided in plasmid vectors containing convenient restriction sites for insertion of a DNA segment of the present invention.

Typical prokaryotic vector plasmids are: pUC18, pUC19, pBR322 and pBR329 available from Biorad Laboratories (Richmond, CA, USA); p*Trc*99A, pKK223-3, pKK233-3, pDR540 and pRIT5 available from Pharmacia (Piscataway, NJ, USA); pBS vectors, Phagescript vectors, Bluescript vectors, pNH8A, pNH16A, pNH18A, pNH46A available from Stratagene Cloning Systems (La Jolla, CA 92037, USA).

A typical mammalian cell vector plasmid is pSVL available from Pharmacia (Piscataway, NJ, USA). This vector uses the SV40 late promoter to drive expression of cloned genes, the highest level of expression being found in T antigen-producing cells, such as COS-1 cells. Examples of an inducible mammalian expression vectors include pMSG, also available from Phannacia (Piscataway, NJ, USA), and the tetracycline (tet) regulatable system, available form Clontech. The pMSG vector uses the glucocorticoid-inducible promoter of the mouse mammary tumour virus long terminal repeat to drive expression of the cloned gene. The tet regulatable system uses the presence or absence of tetracycline to induce protein expression via the tet-controlled transcriptional activator.

Useful yeast plasmid vectors are pRS403-406 and pRS413-416 and are generally available from Stratagene Cloning Systems (La Jolla, CA 92037, USA). Plasmids pRS403, pRS404, pRS405 and pRS406 are Yeast Integrating plasmids (YIps) and incorporate the yeast selectable markers *HIS3, TRP1, LEU2* and *URA3.* Plasmids pRS413-416 are Yeast Centromere plasmids (YCps).

Methods well known to those skilled in the art can be used to construct expression vectors containing the coding sequence and, for example appropriate transcriptional or translational controls. One such method involves ligation via homopolymer tails. Homopolymer polydA (or polydC) tails are added to exposed 3' OH groups on the DNA fragment to be cloned by terminal deoxynucleotidyl transferases. The fragment is then capable of annealing to the polydT (or polydG) tails added to the ends of a linearised plasmid vector. Gaps left following annealing can be filled by DNA polymerase and the free ends joined by DNA ligase.

Another method involves ligation via cohesive ends. Compatible cohesive ends can be generated on the DNA fragment and vector by the action of suitable restriction enzymes. These ends will rapidly anneal through complementary base pairing and remaining nicks can be closed by the action of DNA ligase.

A further method uses synthetic molecules called linkers and adaptors. DNA fragments with blunt ends are generated by bacteriophage T4 DNA polymerase or *E. coli* DNA polymerase I which remove protruding 3' termini and fill in recessed 3' ends. Synthetic linkers, pieces of blunt-ended double-stranded DNA which contain recognition sequences for defined restriction enzymes, can be ligated to blunt-ended DNA fragments by T4 DNA ligase. They are subsequently digested with appropriate restriction enzymes to create cohesive ends and ligated to an expression vector with compatible termini. Adaptors are also chemically synthesised DNA fragments which contain one blunt end used for ligation but which also possess one pre-formed cohesive end.

Synthetic linkers containing a variety of restriction endonuclease sites are commercially available from a number of sources including International Biotechnologies Inc, New Haven, CN, USA.

A desirable way to modify the nucleic acid molecule encoding the transglutaminase is to use the polymerase chain reaction as disclosed by Saiki *et al.* (1988). In this method the nucleic acid molecule, *e.g*. DNA, to be enzymatically amplified is flanked by two specific oligonucleotide primers which themselves become incorporated into the amplified DNA. The said specific primers may contain restriction endonuclease recognition sites which can be used for cloning into expression vectors using methods known in the art.

Conveniently, the transglutaminase is a variant transglutaminase.

By "a variant" we include a polypeptide comprising the amino acid sequence of a naturally occurring transglutaminase wherein there have been amino acid insertions, deletions or substitutions, either conservative or non-conservative, such that the changes do not substantially reduce the activity of the variant compared to the activity of the activated naturally occurring trans glutaminase. For example, the variant may have increased crosslinking activity compared to the crosslinking activity of the naturally occurring transglutaminase.

The enzyme activity of variant transglutaminases may be measured by the biotin-cadaverine assay, as described in the Examples and as published in (Jones *et al.,* 1997). For example, reduced expression of tissue transglutaminase in a human endothelial cell line leads to changes in cell spreading, cell adhesion and reduced polymerisation of fibronectin. Alternatively, transglutaminase activity may be measured by the incorporation of [¹⁴C]-putrescine incorporation into N,N'-dimethylcasein, as outlined by Lorand *et al.,* 1972. The increased ability of the variant enzyme to facilitate the adhesion and spreading of cells on medical implants may be measured by the methods disclosed herein.

Variant transglutaminases may be made using methods of protein engineering and site-directed mutagenesis commonly known in the art (for example, see Sambrook & Russell, *supra.*)*.*

Advantageously, the variant transglutaminase is a fragment of a naturally occurring transglutaminase which retains the ability of said naturally occurring transglutaminase to promote collagen crosslinking.

It will be appreciated that in the invention, the treatment of the collagen-containing starting material with a transglutaminase must be performed under conditions which allow the formation of ε-(γ-glutamyl) lysine crosslinks in the collagen. Such conditions may readily be determined by persons skilled in the art For example, the formation of ε-(γ-glutamyl) lysine crosslinks may be measured as described in the Examples below.

Preferably, the collagen starting material is neutralised prior to treatment with the transglutaminase (in order to facilitate collagen fibril formation and to promote transglutaminase activity).

Advantageously, the transglutaminase is used at a concentration of between 50 and 1000 µg per ml of reaction mixture. Preferably, the collagen conentration within the reaction mixture is 3 to 6 mg/ml.

The crosslinking reaction mixture containing the collagen and the transglutaminase may further comprise one or more of the following:
(i) a reducing agent (for example, DTT);
(ii) calcium ions (for example, CaCl₂); and/or
(iii) a buffering agent which buffers the reaction mixture at pH 7.4.

Preferably, treatment with the transglutaminase is performed at 37°C.

Also described herein is a biomaterial comprising crosslinked collagaen obtained or obtainable by a use according to the invention.

Preferably, the biomaterial is substantially free of catalysts, initiators and/or unreacted or partially reacted crosslinking agents, wherein the unreacted or partially reacted crosslinking, agent is not a transglutaminase.

Additionnally described herein is the use of a biomaterial as described above in the manufacture of a medical implant or wound dressing.

Furtther described herein is a medical implant comprising a biomaterial as described above. Preferably, the medical implant material is artificial bone.

It will be appreciated that the medical implant may consist solely of a biomaterial of the invention or, alternatively, may comprise a biomaterial of the invention together with one or more other biomaterials. For example, the medical implant may comprise a biomaterial of the invention which is coated, impregnated covalently linked or otherwise mixed with a known biomaterial, such as metals, bioceramics, glass or biostable polymers (for example polyethylene, polypropylene, polyurethane, polytetrafluoroethylene, poly(vinyl chloride), polyamides, poly(methylmethacrylate), polyacetal, polycarbonate, poly(-ethylene terphthalate), polyetheretherketone, and polysulfone).

Also described herein is a wound dressing comprising a biomaterial as described above.

The medical implants and wound dressings may take the form of a sponge or a freeze-dried lattice after TGase crosslinking, or may easily be made in a variety of ways (see below).

| FORM OF COLLAGEN | APPLICATIONS |
|---|---|
| Fibres | Suture material, weaving blood vessels, valve prosthesis, haemostatic fleece, knitted or woven fabric as tissue support |
| Flour or powder | Haemostatic agent |
| Film, membrane or tape | Corneal replacement, contact lens, haemodialysis, artificial kidneys, membrane oxygenators, wound dressing; patches (aneurism, bladder, hernia) |
| Gel | Vitreous body, cosmetics (creams) |
| Solution | Plasma expander, vehicle for drug delivery system, injectable in skin and lip cosmetic defects |
| Sponge or felt | Wound dressing, bone-cartilage substitute, surgical tampons, laparatomy pads, contraceptives, vessel prosthesis, reservoir for drug delivery |
| Tubing | Reconstructive surgery of hollow organs (oesophagus, trachea) |

| | |
|---|---|
| Taken from: Chvapil, 1979. In Fibrous Proteins: Scientific, Industrial and MedicalAspects Vol.1, 4th International Conference on Fibrous Proteins (Massey University) (Editors: Parry DAD and Creamer LK). London Academic Press. p259 | |

In a preferred embodiment, the medical implants and wound dressings are provided in a sealed package. Preferably, the package is sterile. Methods of producing such packages are well known in the art.

Also described herein is a kit for producing a biomaterial as described above comprising collagen, a transglutaminase and, optionally, a cell adhesion factor (such as fibronectin).

In a preferred embodiment, the kit is provided in a sealed package. Preferably, the package is sterile.

Advantageously, the kit further comprises instructions for performing a method according to the first aspect of the invention.

The invention will now be described in detail with reference to the following figures and examples:

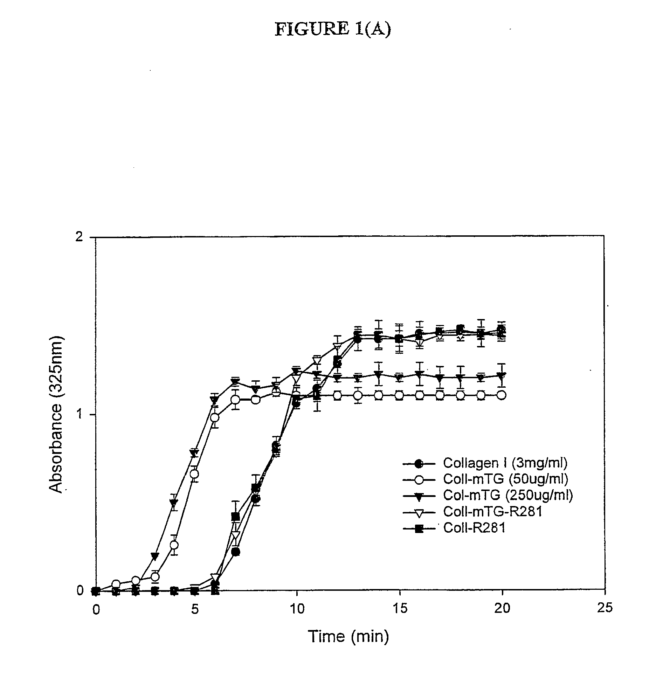

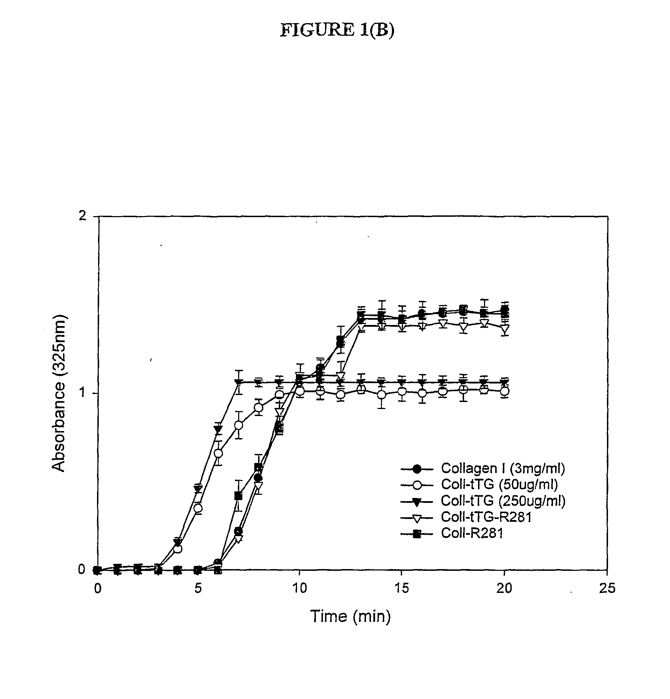

**Figure 1**. Type I collagen fibrillogenesis after neutralisation in the presence of transglutaminases. Collagen (3mg/ml) was neutralised as in the methods and was treated with 0, 50 or 250µg/ml of microbial TG (A) or tTG (B). 500 µM of the TGase inhibitor *N-*Benzyloxycarbonyl-*L-*phenylalanyl-6-dimethyl-sulfonium-5-oxo-*L-*norleucine ('R281') was used to confirm that the effects were due to transglutaminase activity. The absorbance at 325nm was measured using a PYE Unicam SP 1800 UV spectrophotometer. The temperature was controlled at 25°C using a Techne C-85A circulator. Results are from the average of 3 independent experiments.

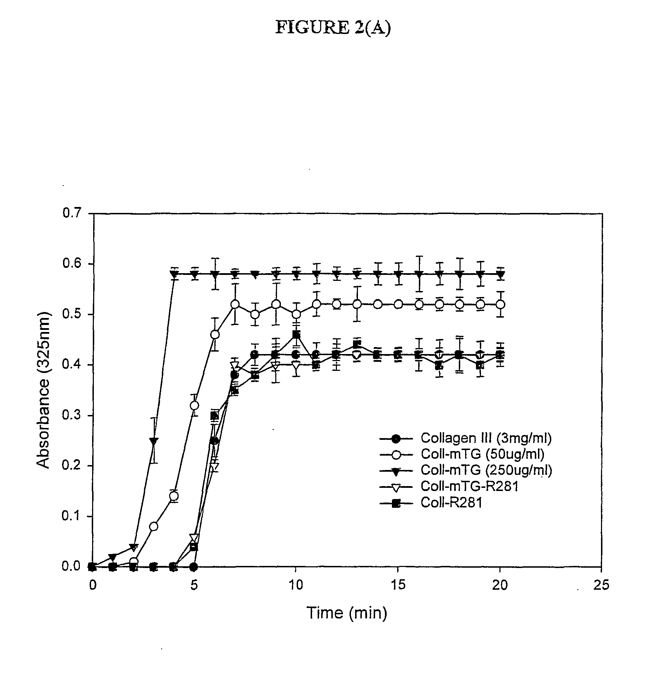

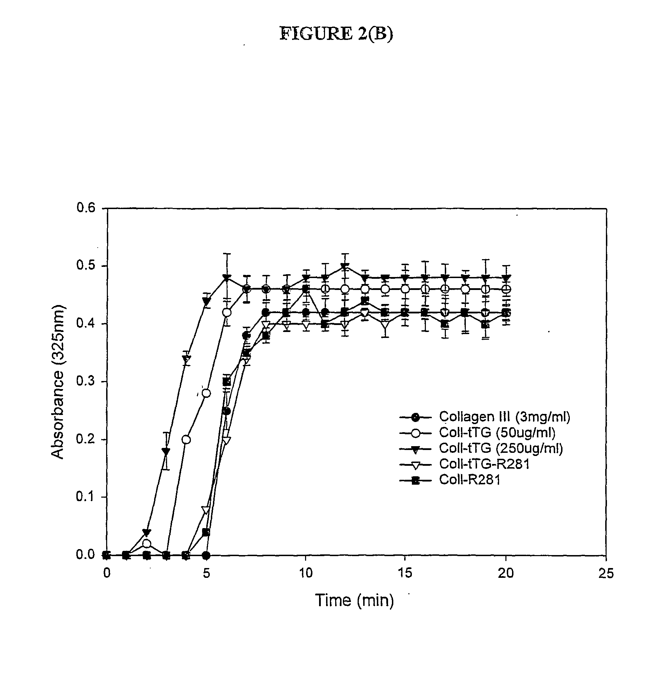

**Figure 2**. Type III collagen fibrillogenesis after neutralisation in the presence of transglutaminases. Collagen (3mg/ml) was neutralised as in the methods and was treated with 0, 50 or 250µg/ml of mTG (A) or tTG (B). 500 µM inhibitor R281 was used to confirm that the effects were due to transglutaminase activity. The absorbance at 325nm was measured using a PYE Unicam SP 1800 UV spectrophotometer. The temperature was controlled at 25°C using a Techne C-85A circulator. Results are from the average of 3 independent experiments.

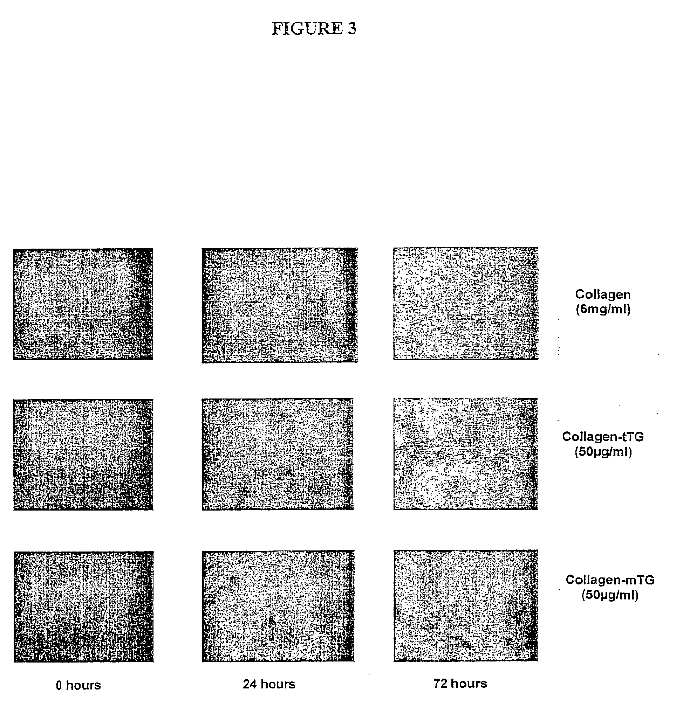

**Figure 3**. HOB cell mediated collagen degradation monitored using Coomassie blue staining. Collagen (6mg/ml) was pre-treated with either 50µg/ml tTG or mTG (activities: tTG: 11500 Units/mg; mTG: 16000 Units/mg) using the incorporation technique. HOB cells were then seeded at 2000 cells/well onto the different substrates, using complete media, in a humidified-atmosphere incubator at 37°C with 5% CO₂. At the relevant time points, the cells were removed and the substrates washed twice with PBS and distilled water. Samples were then stained using 0.1% Coomassie brilliant blue stain solution. Pictures were then taken using an Olympus microscope and digital camera under x400 magnification.

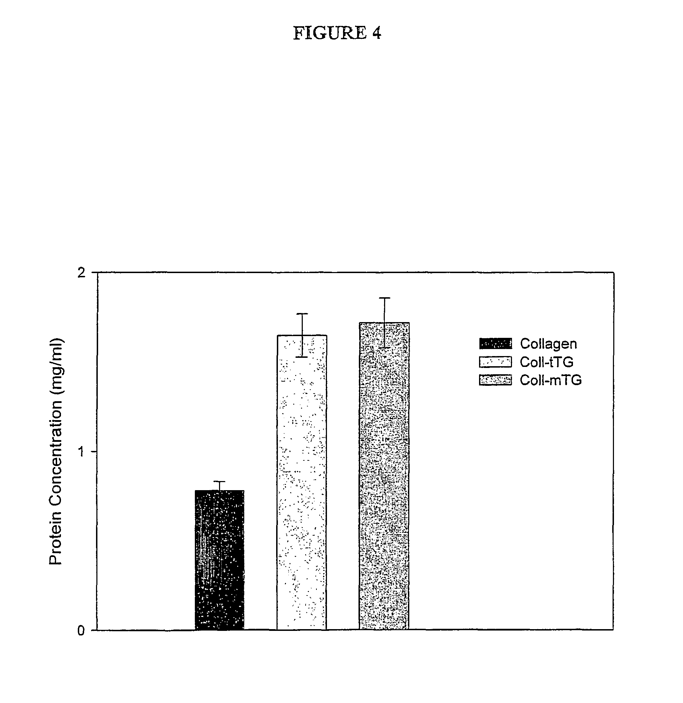

**Figure 4**. Residual protein concentration (after 72 hours) of native and TG-treated collagen gels following the culture of HOB cells. Collagen (6mg/ml) was pre-treated with either 50µg/ml tTG or mTG (activities: tTG: 11500 Units/mg; mTG: 16000 Units/mg). HOB cells were then seeded at 2000 cells/well onto the different substrates, using complete media, in a humidified-atmosphere incubator at 37°C with 5% CO₂. After 72 hours, the cells were removed and the residual collagen-substrates, if any, were washed twice with PBS and distilled water. Further treatment with microbial collagenase and trypsin for 24 hours was performed as described in the Materials and Methods section. The protein concentrations of these were determined by the Lowry assay. Results are from three independent experiments, each with triplicate samples, and are expressed as mean values with ±SD.

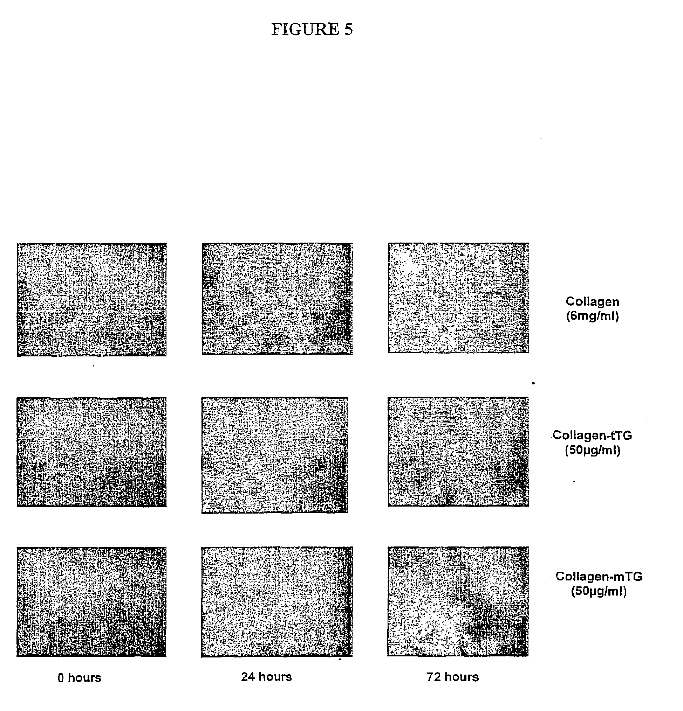

**Figure 5**. HFDF cell mediated collagen degradation monitored using Coomassie blue staining. Collagen (6mg/ml) was pre-treated with either 50µg/ml tTG or mTG (activities: tTG: 11500 Units/mg; mTG: 16000 Units/mg) using the incorporation technique. HFDF cells were then seeded at 2000 cells/well onto the different substrates, using complete media, in a humidified-atmosphere incubator at 37°C with 5% CO₂. At the relevant time points, the cells were removed and the substrates washed twice with PBS and distilled water. Samples were then stained using 0.1 % Coomassie brilliant blue stain solution. Pictures were then taken using an Olympus microscope and digital camera under x400 magnification.

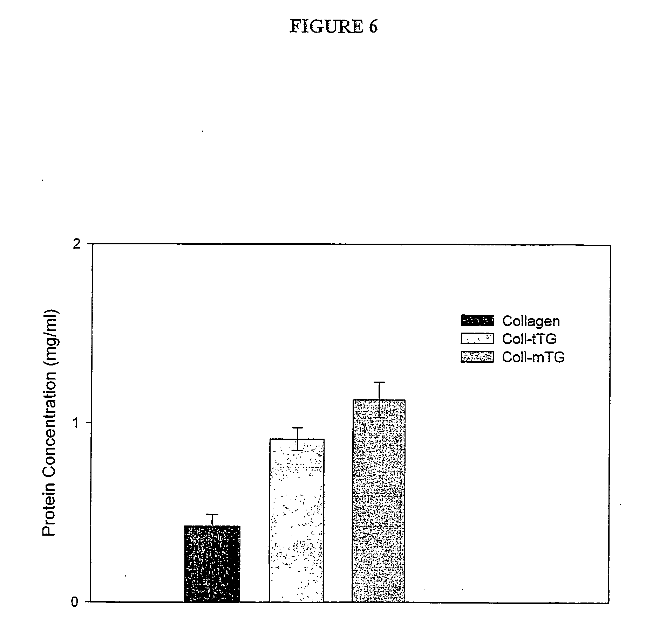

**Figure 6**. Residual protein concentration (after 72 hours) of native and TG-treated collagen gels following the culture of HFDF cells. Collagen (6mg/ml) was pre-treated with either 50µg/ml tTG or mTG (activities: tTG: 11500 Units/mg; mTG: 16000 Units/mg). HFDF cells were then seeded at 2000 cells/well onto the different substrates, using complete media, in a humidified-atmosphere incubator at 37°C with 5% CO₂. After 72 hours, the cells were removed and the residual collagen-substrates, if any, were washed twice with PBS and distilled water. Further treatment with microbial collagenase and trypsin for 24 hours was performed as described in the Materials and Methods section. The protein concentrations of these were determined by the Lowry assay. Results are from three independent experiments, each with triplicate samples, and are expressed as mean values with ±SD.

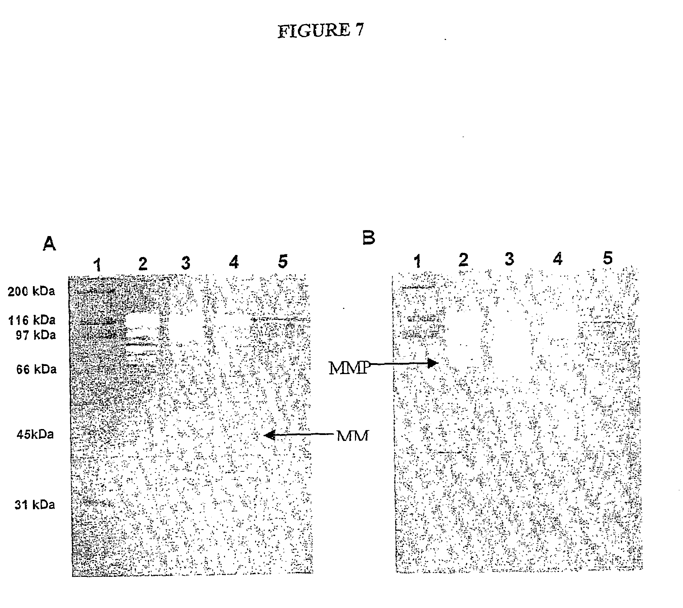

**Figure 7**. Collagen (A) and gelatin (B) zymography of HFDF cell culture supernatants after 24h growth on different media. Lane 1: molecular weight markers (BioRad 161-0317); lane 2: supernatant after growth on GPL tTG treated collagen; lane 3: supernatant after growth on mTG treated collagen; lane 4: supernatant after growth on untreated collagen; lane 5: supernatant after growth in the absence of collagen.

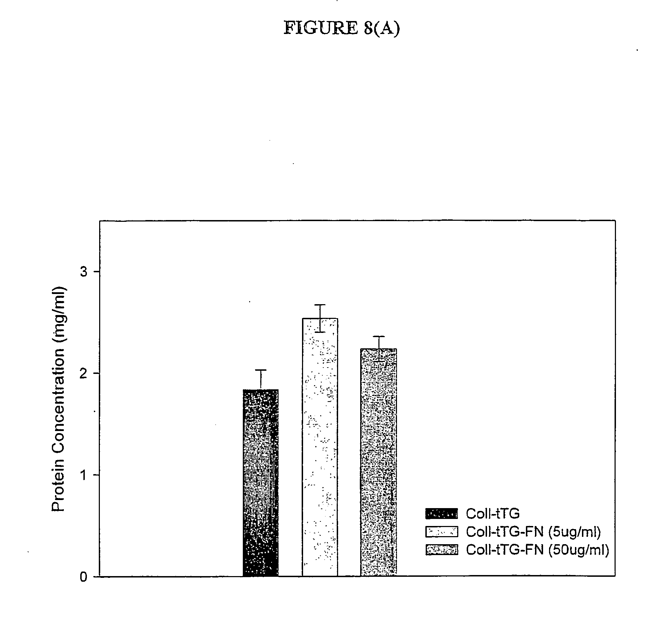

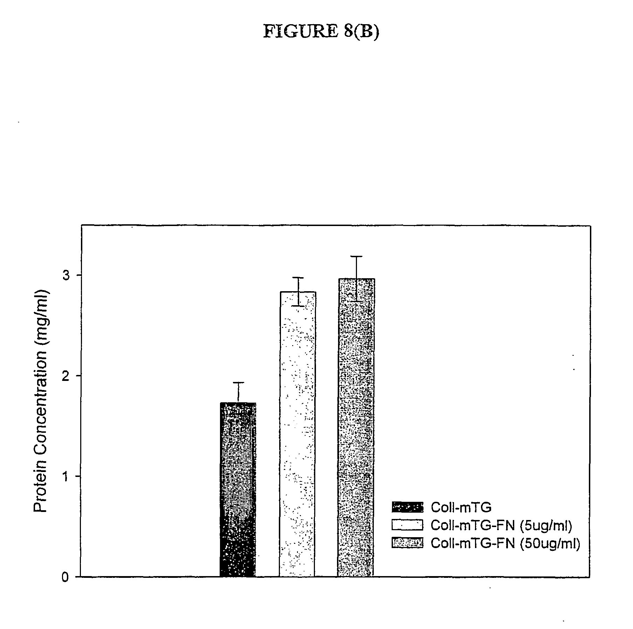

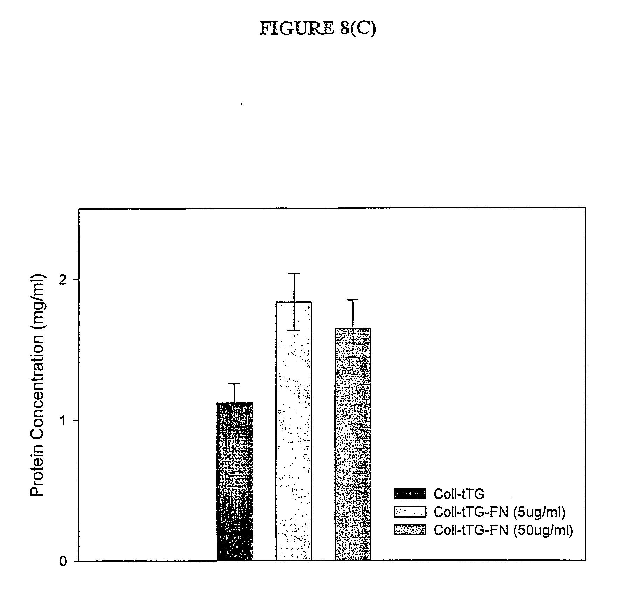

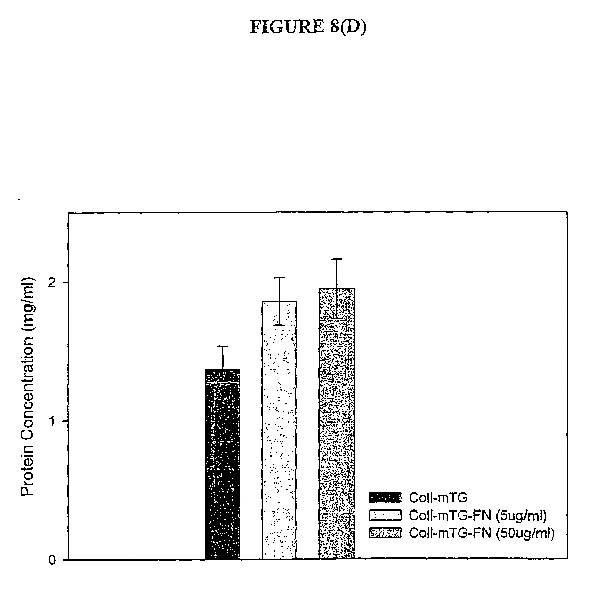

**Figure 8**. Residual protein concentration (after 72 hours) of cross-linked and fibronectin-incorporated collagen gels following culture of HOB and HFDF cells. Collagen (6mg/ml), incorporated with 5µg/ml or 50µg/ml of fibronectin, was pre-treated with either 100µg/ml tTG or mTG (activities: tTG: 11500 Units/mg; mTG: 16000 Units/mg). HOB (Figures 5A and 5B) and HFDF (Figures 5C and 5D) cells were then seeded at 2000 cells/well onto the different substrates, using complete media, in a humidified-atmosphere incubator at 37°C with 5% CO₂. After 72 hours, the cells were removed and the residual collagen-substrates, if any, were washed twice with PBS and distilled water. Further treatment with microbial collagenase and trypsin for 24 hours was performed as described in the Materials and Methods section. The protein concentrations of these were determined by the Lowry assay. Results are from two independent experiments, each with triplicate samples, and are expressed as mean values with ±SD.

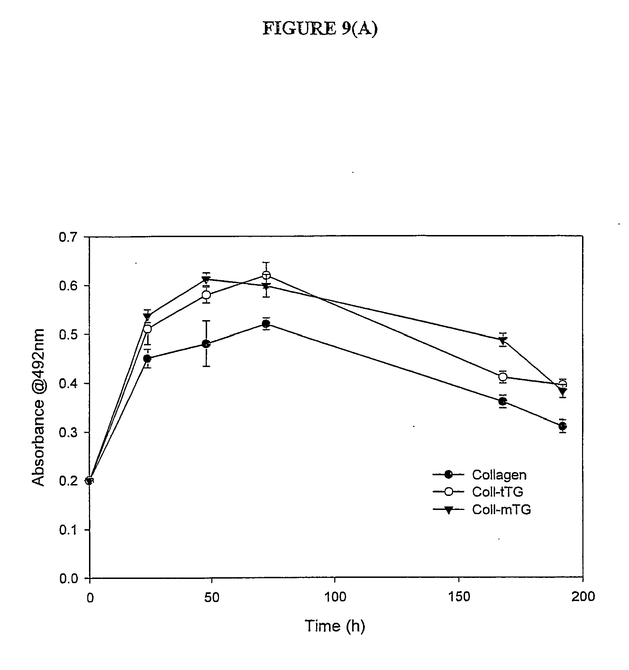

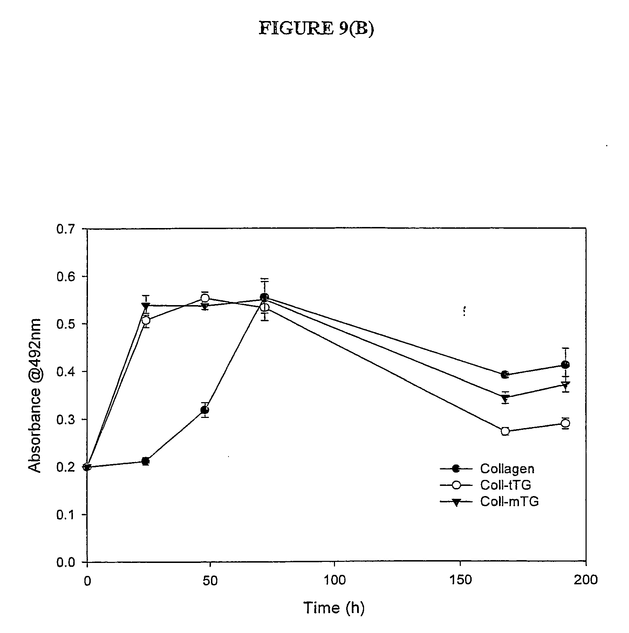

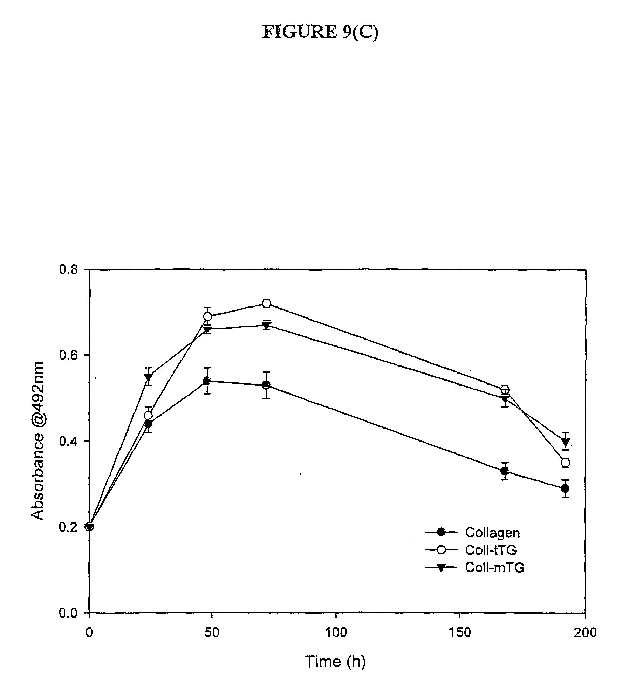

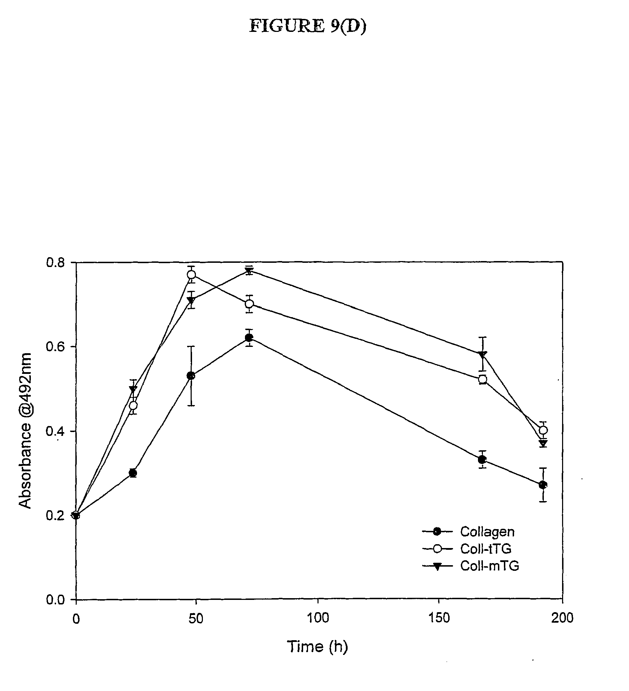

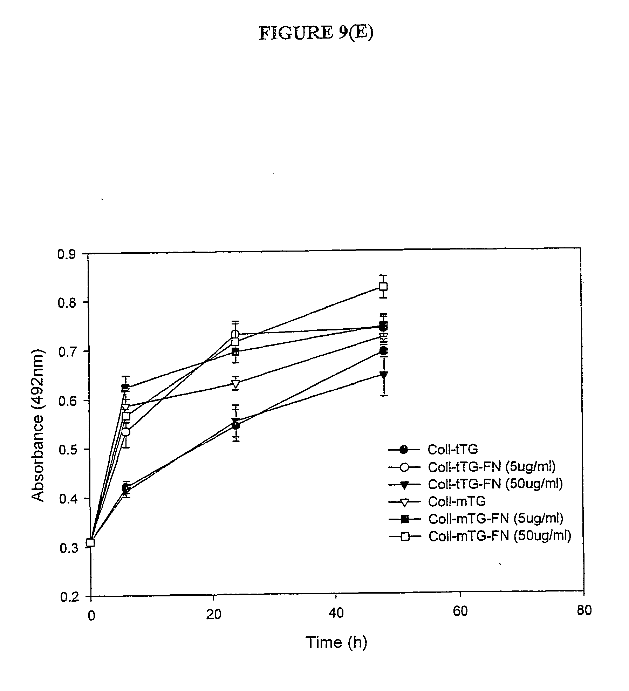

**Figure 9**. Proliferation of HOB and HFDF cells when cultured on native and TG-treated collagen substrates (6A and 6B correspond to 50µg/ml of TG; 6C to 6F corresponds to 100µg/ml of TG). Collagen (6mg/ml) was pre-treated with either a combination of 50 or 100µg/ml tTG, 50 or 100µg/ml of mTG, or 5 or 50µg/ml of fibronectin (activities: tTG: 11500 Units/mg; mTG: 16000 Units/mg). HOB (Figures 9A, 9C and 9E) and HFDF (Figures 9B, 9D and 9F) cells were initially seeded at 2000 cells/well of a 96 well plate and cultured on the different substrates, using complete media, in a humidified-atmosphere incubator at 37°C with 5% CO₂, for the relevant time points. Proliferation rates were determined by treatment of the samples with CellTiter AQ solution as described in the Materials and Methods section. Results represent the mean value and ±SD from four independent experiments, each having triplicate samples.

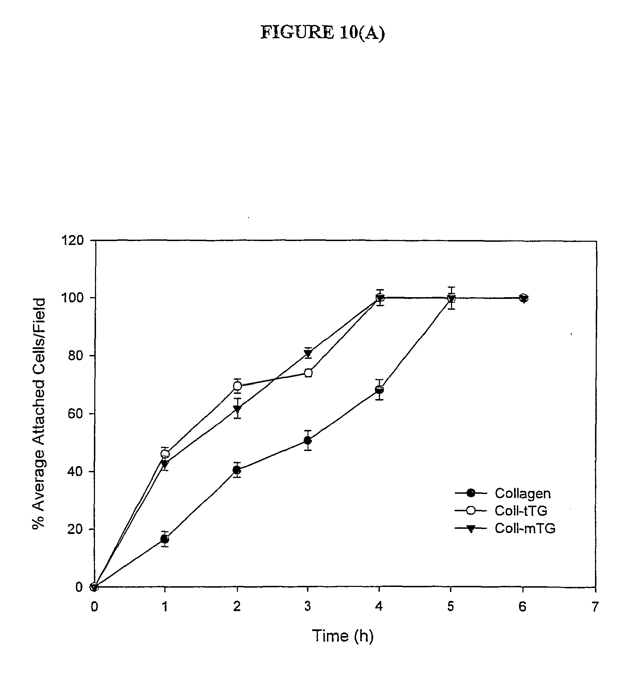

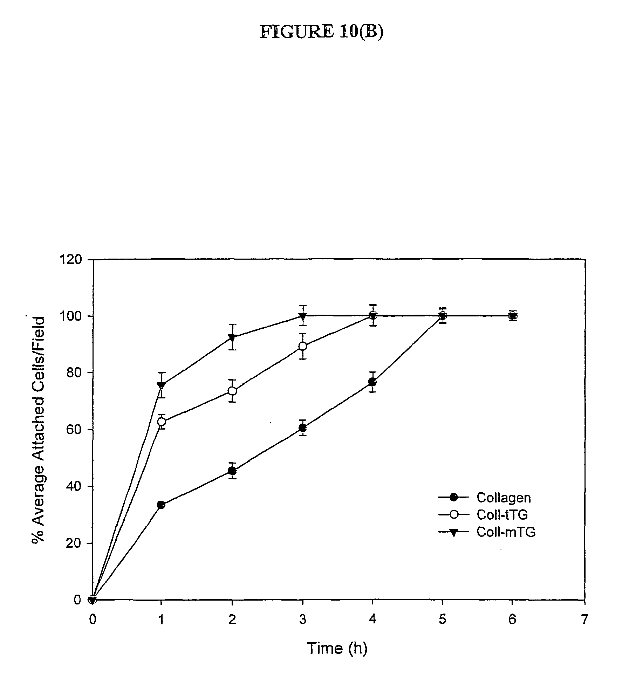

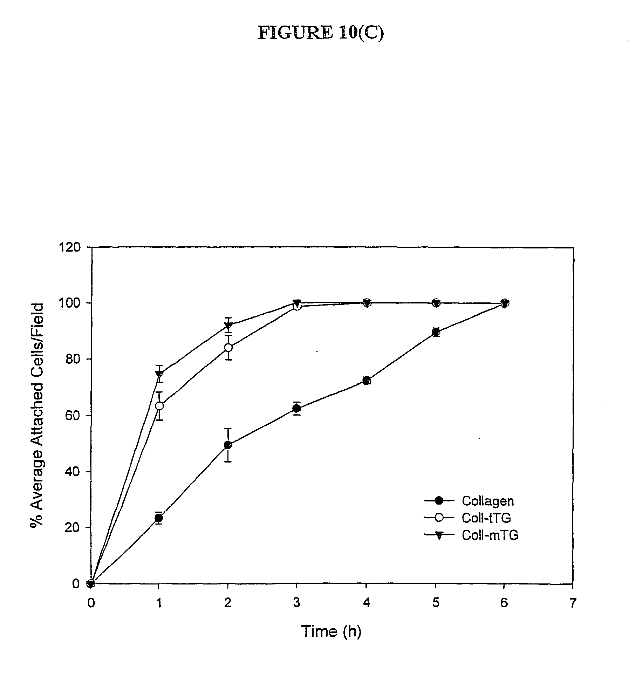

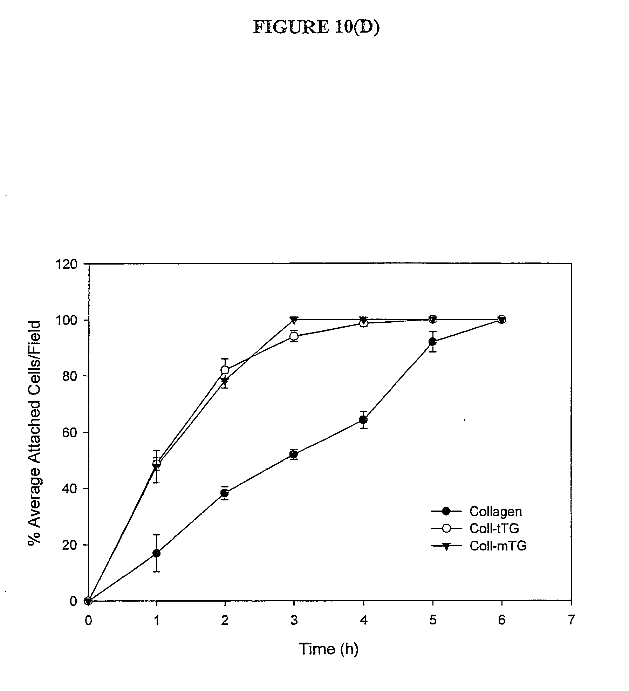

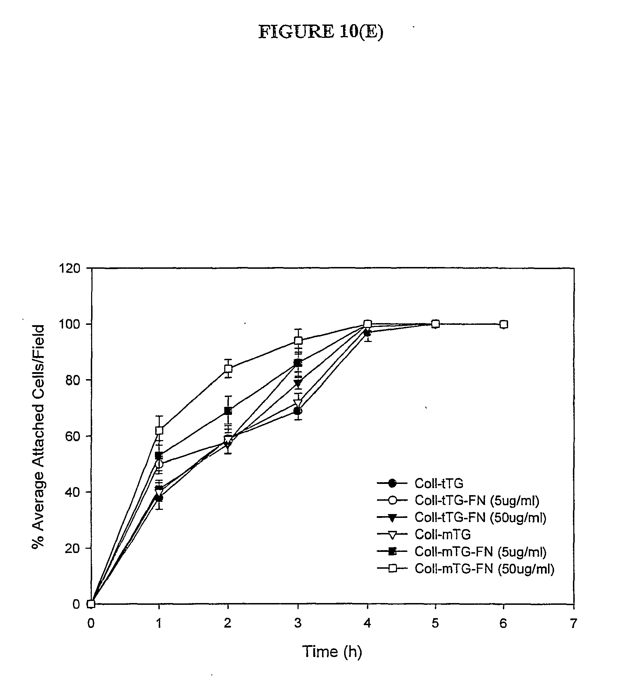

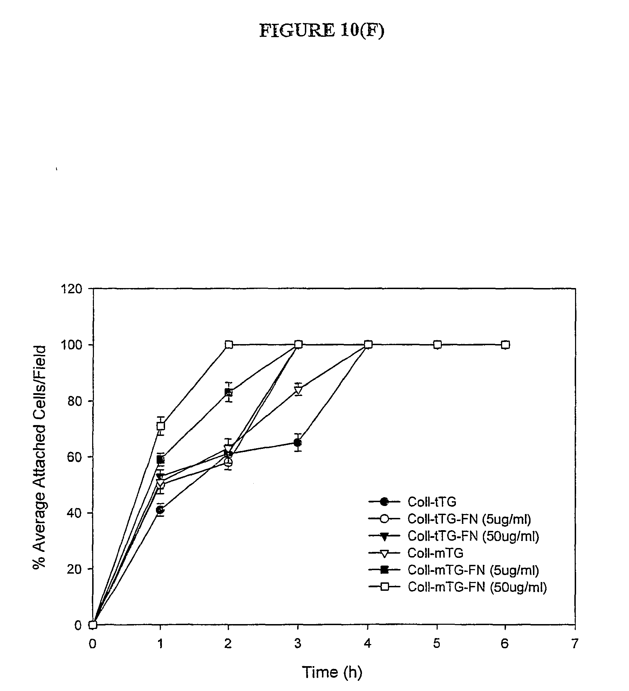

**Figure 10**. Attachment characteristics of HOB and HFDF cells on native, TG-treated and TG-FN incorporated collagen substrates (10A and 10B correspond to 50µg/ml of TG; 10C to 10F corresponds to 100µg/ml of TG). Collagen (6mg/ml) was pre-treated with either a combination of 50 or 100µg/ml tTG, 50 or 100µg/ml of mTG, or 5 or 50µg/ml of fibronectin (activities: tTG: 11500 Units/mg; mTG: 16000 Units/mg). HOB (Figures 10A, 10C and 10E) and HFDF (Figures 10B, 10D and 10F) cells were then initially seeded at 2000 cells/well of a 96 well plate and cultured on the different substrates, using complete media, in a humidified-atmosphere incubator at 37°C with 5% CO₂, for the relevant time points.
Cells were fixed using 3.7% (w/v) paraformaldehyde before being stained with May-Grunwald and Giemsa stains and then viewed under a light microscope. Pictures were then taken and attached cells analysed using ScionImage™ software. Results are from four independent experiments,
each with triplicate samples, and are expressed as mean values with ±SD.
Attachment characteristics are represented by the (% average attached cells per field) derived from the attached average cells divided by total attached cells at 6hours. The field of vision corresponds to the visible area observed at an x400 magnification with cell numbers ranging from 50-100 cells per field.

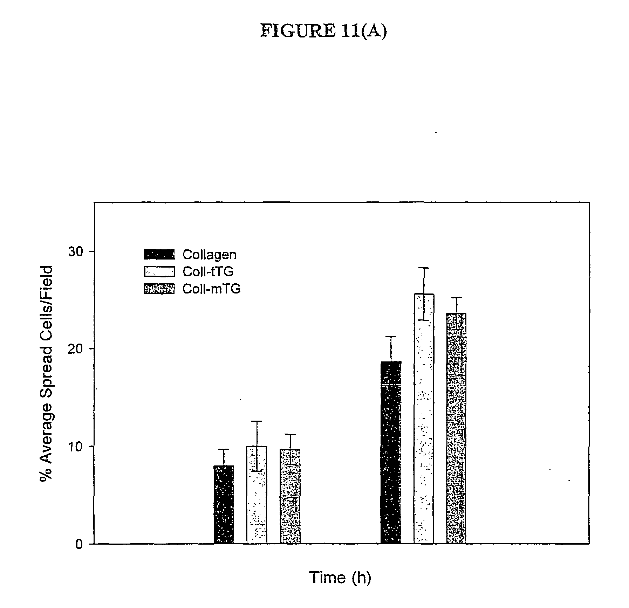

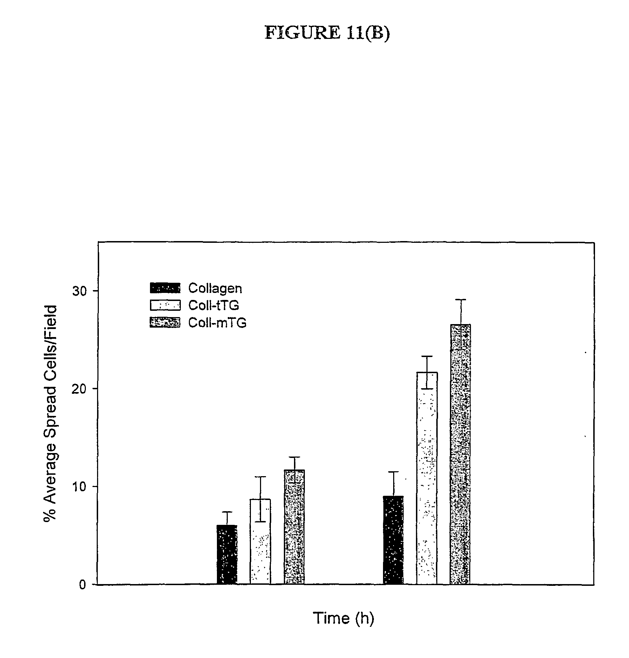

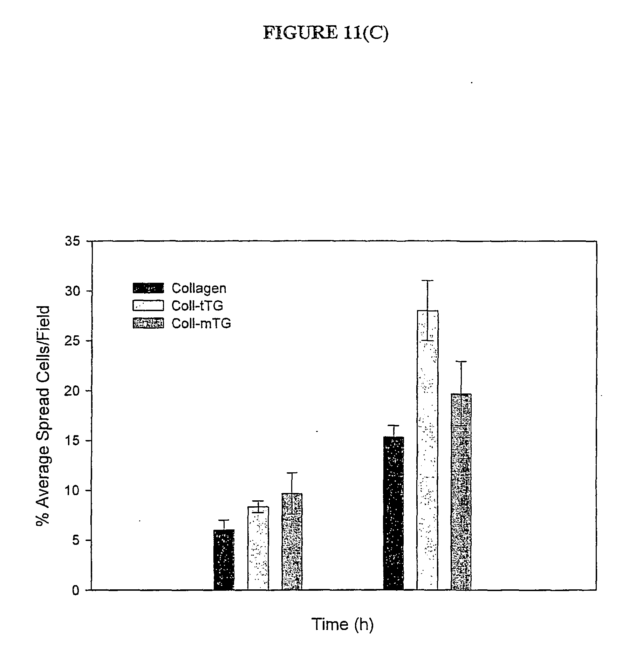

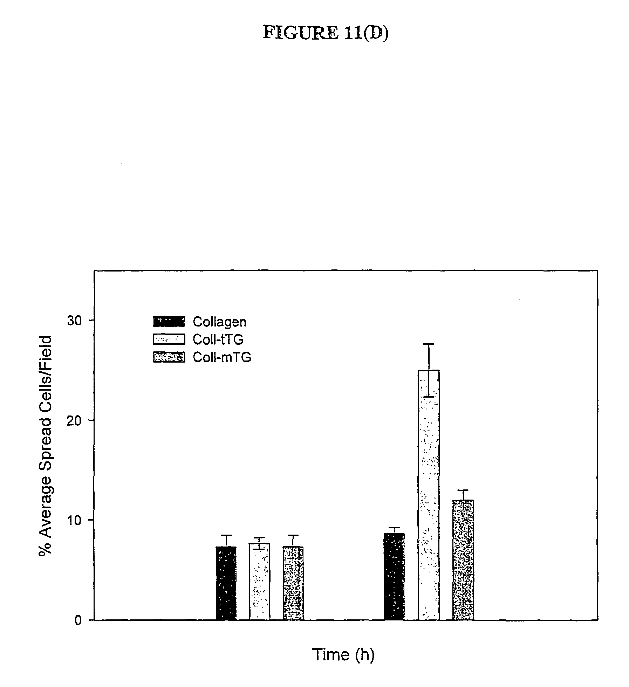

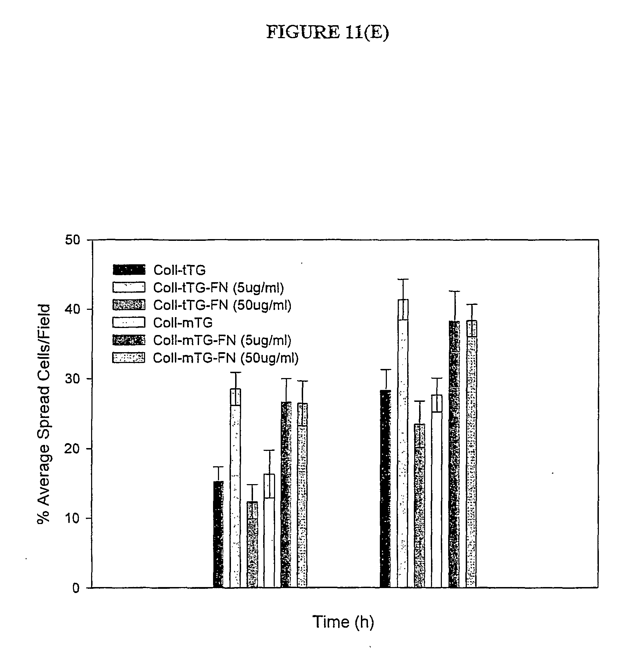

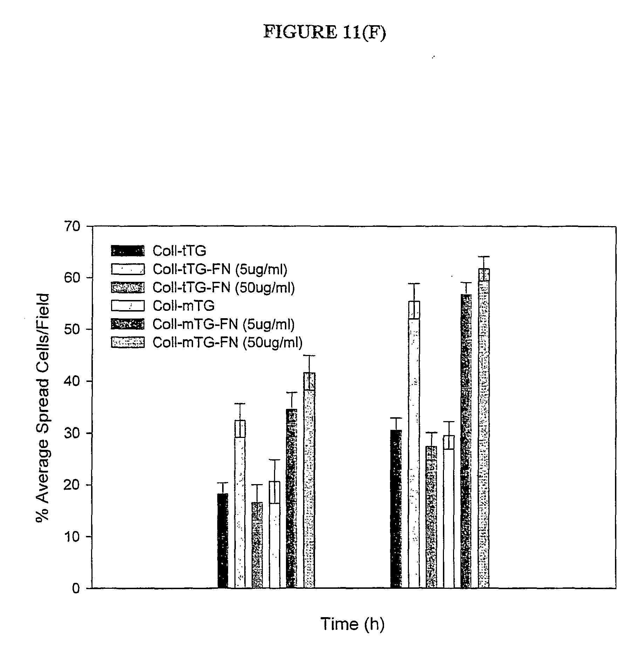

**Figure 11**. Spreading characteristics of HOB and HFDF cells on native, TG-treated and TG-FN incorporated collagen substrates (11A and 11B correspond to 50µg/ml of TG; 8C to 11F corresponds to 100µg/ml of TG). Collagen (6mg/ml) was pre-treated with either a combination of 50 or 100µg/ml tTG, 50 or 100µg/ml of mTG, or 5 or 50µg/ml of fibronectin (activities: tTG: 11500 Units/mg; mTG: 16000 Units/mg). HOB (Figures 11A, 11C and 11E) and HFDF (Figures 11B, 11D and 11F) cells were then initially seeded at 2000 cells/well of a 96 well plate and cultured on the different substrates, using complete media, in a humidified-atmosphere incubator at 37°C with 5% CO₂, for the relevant time points.
Cells were fixed using 3.7% (w/v) paraformaldehyde before being stained with May-Grunwald and Giemsa stains and then viewed under a light microscope. Pictures were then taken and spread cells analysed using ScionImage™ software. Where by attached and spread cells were distinguished and characterised based upon the deviations of their cytoplasm- as previously described by Jones et al., (1997). Results are from four independent experiments and represent the 1-hour and 6-hour time points respectively. Each experiment is with triplicate samples, and are expressed as mean values with ±SD. Spreading characteristics are represented by the (% average spread cells per field) derived from the average spread cells divided by total cells in the field of vision. The field of vision corresponds to the visible area observed at an x400 magnification with cell numbers ranging from 50-100 cells per field.

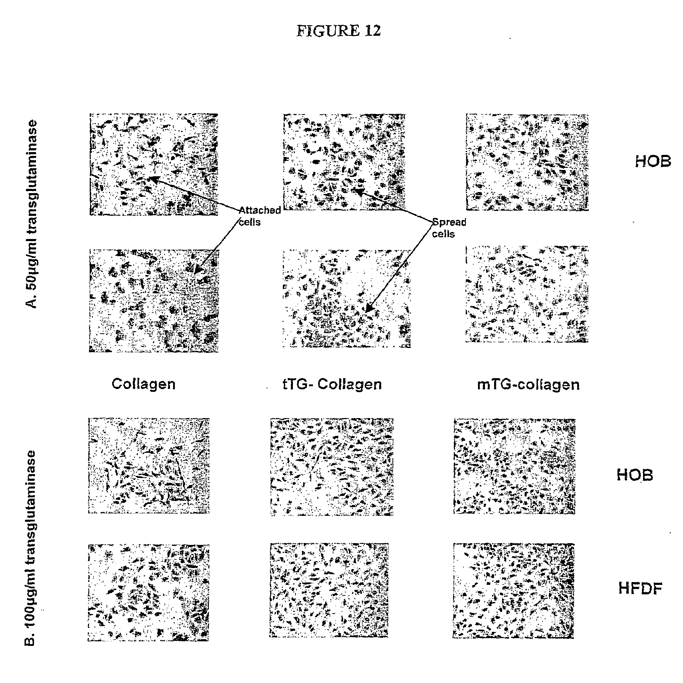

**Figure 12**. Attachment and spreading characteristics of HOB cells on tTG-treated collagen. Collagen (6mg/ml) was pre-treated with either tTG or mTG at 50-100µg/ml (activities: tTG: 11500 Units/mg; mTG: 16000 Units/mg). HOB and HFDF cells were then initially seeded at 2000 cells/well of a 96 well plate and cultured on the different substrates, using complete media, in a humidified-atmosphere incubator at 37°C with 5% CO₂, for the relevant time points. Cells were fixed using 3.7% (w/v) paraformaldehyde before being stained with May-Grunwald and Giemsa stains and then viewed with an Olympus C2 microscope before pictures were taken with an Olympus DP10 digital camera. Figures are from a field of vision, under x400 magnification, and indicate the 6 hour time point with cell numbers ranging from 50-100 cells per field.

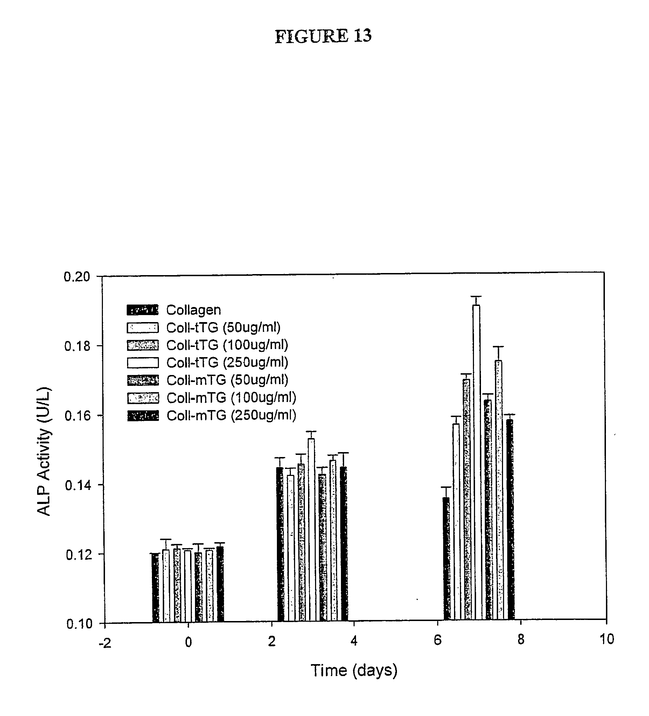

**Figure 13**. Alkaline phosphatase activity of HOB cells cultured on TG-treated collagen substrates. Collagen (6mg/ml) was pre-treated with either 50-250µg/ml tTG or mTG (activities: tTG: 11500 Units/mg; mTG: 16000 Units/mg). Cells were initially seeded at 2000 cells/well of a 96 well plate and cultured on the different substrates, using complete media, in a humidified-atmosphere incubator at 37°C with 5% CO₂, for the relevant time points. 50µl of combined triplicate supernatant samples were taken and the ALP levels determined using the ALP Optimized Alkaline Phosphatase EC3131 Colorimetric kit (Sigma) as described in the Materials and Methods section. Results represent the mean value and ±SD from three independent experiments.

### EXAMPLES

### Méthods and Materials

All water used was de-ionised using an Elgastat System 2 water purifier (ELGA Ltd. UK) and a Milli-Q water purifier (Millipore Waters, UK). All chemicals were purchased from Sigma-Aldrich, Poole, UK, unless otherwise stated. Sterile preparation of stock solutions and chemicals were performed either by filtration through a 0.22µm Whatmann sterile filter and/or autoclaving at 121°C at 15psi for 1h. Centrifuges and other handling equipment were cleaned with 70% ethanol prior to use.

### Cell culture

Human osteoblast (HOB) cells, isolated from explants of trabecular bone dissected from femoral heads following orthopaedic surgery, as described by DiSilvio (1995) were kindly supplied by Professor S. Downes and Dr. S. Anderson (School of Biomedical Sciences, University of Nottingham) and used during this investigation. Human foreskin dermal fibroblast (HFDF) cells isolated from human neonatal foreskin (Mr. P. Kotsakis, School of Science, Nottingham Trent University) were also used. Both cell lines were used during their low-passage number; ranging from between 11 to 25 passages. Cell lines were cultured and maintained, in vitro, as monolayers in T-flasks using DMEM, supplemented with 10% heat-inactivated (56°C for 1h) FCS, 1% non-essential amino acids and 2mM L-glutamine. Periodic additions of 1% penicillin-streptomycin were used to avoid bacterial contamination. Flasks were kept in a humidified-atmosphere incubator at 37°C and with 5% CO₂. Cells were routinely passaged and allowed to reach greater than 90% confluency at any one time. For detachment, standard trypsinisation was performed using 0.25% (w/v) trypsin/2mM EDTA solution in PBS solution.

### Cell Viability and Proliferation

Cell counts and viability estimations were performed using the standard trypan blue exclusion technique by means of a 0.22µm sterile filtered 0.5% (w/v) trypan blue solution and a haemocytometer. Non-viable cells stained blue due to the loss of their membrane integrity and, hence, allowed the passage of dye into the cell. Viable cells remained colourless.

Cell proliferation and viability were also measured using the CellTiter AQ One Solution Cell Proliferation™ assay kit (Promega, Southampton, UK. Cat no. G3580). This reagent contains a novel tetrazolium compound (MTS) and an electron coupling reagent (PES). The MTS tetrazolium compound is bioreduced by cells into a coloured formazan product that is soluble in tissue culture medium. This conversion is accomplished by NADPH or NADH produced by dehydrogenase enzymes in metabolically active cells. Assays were performed, in the dark, simply by the addition of 20µl of CellTiter AQ reagent into the relevant samples in 100µl of culture medium. These samples were then incubated in a humidified-atmosphere incubator at 37°C and with 5% CO2 for 90 minutes before the absorbance was read at 490nm using a SpectraFluor® plate reader.

### Attachment and Spreading

Cells were seeded on the relevant substrate at a density of 625 cells/mm². After allowing cells to proliferate, they were fixed in 3.7% (w/v) paraformaldehyde, permeabilised by the addition of 0.1 % (v/v) Triton X-100 in PBS, before staining with May-Grunwald (0.25% (w/v) in methanol) and Giemsa stains (0.4% (w/v) in methanol, diluted 1:50 with water). Cells were then viewed under a x400 magnification using an Olympus CK2 microscope. Three separate fixed-size random fields per sample were photographed with an Olympus DP 10 digital camera. Pictures were analysed using Scion Image™ software (Scion Corporation, Maryland, USA) whereby attached and spread cells were distinguished and characterised based upon the deviations of their cytoplasm- as previously described by Jones et al., (1997).

### Alkaline Phosphatase Activity

The ALP Optimized Alkaline Phosphatase EC 3.1.3.1 Colorimetric Test® kit (obtained from Sigma-Aldrich, Poole, UK. Cat no. DG1245-K) was used to quantify the ALP activity. Serum ALP hydrolyses p-nitrophenyl phosphate to *p*-nitrophenol and inorganic phosphate. The hydrolysis occurs at alkaline pH and the *p*-nitrophenol formed shows an absorbance maximum at 405nm. The rate of increase in absorbance at 405nm is directly proportional to ALP activity in the sample. Samples were treated according to the manufacturers' instructions and analysed using a Beckmann DU530 UV/Vis Spectrophtometer.

### Transglutaminase

Tissue transglutaminase (tTG) was isolated and purified from guinea pig livers following a modification of the Leblanc et al. (1999) involving both anion exchange, gel filtration and affinity chromatography. Commercial samples of TG were also used during this investigation: tTG from guinea pig liver (Sigma-Aldrich, Poole, UK. Cat no. T5398) and microbial transglutaminase, mTG, (Ajinomoto Corporation Inc. Japan), isolated from Streptoverticillium mobaraenase, as the commercially available product, Activa™ WM. This required further purification steps to remove the incorporated maltodextrin: briefly, the Activa™ WM was dissolved in ice-cold 20mM phosphate buffer, 2mM EDTA pH 6.0 and filtered, before being loaded onto a 100ml SP-Sepharose FF column overnight at a flow rate of 5ml/min by recycling. The column was then washed and proteins eluted with a 0-1000mM gradient of NaCl in 20mM phosphate buffer, 2mM EDTA pH 6.0 over 80min, collecting 5ml fractions. Fractions were assayed for protein using the Bio-Rad DC protein assay (Bio-Rad Laboratories, Hertfordshire, UK. Cat no. 500-0120)- a modification of the Lowry method (Lowry et al., 1951). Fractions containing mTG were pooled, aliquoted, freeze dried and stored at -70°C. Before immediate use, tTG was pre-treated in 2mM DTT in 50mM Tris buffer (pH 7.4) for 10 minutes at room temperature, before addition to a final buffered solution containing 5mM CaCl2 and, a minimum of 1mM DTT in Tris buffer. Typical activities for the transglutaminases used in this investigation were as follows: tTG: 11500-13000 Units/mg and mTG: 16000-25000 Units/mg.

### Transglutaminase Activity

The incorporation of [14-C]-putrescine into N,N'-dimethylcasein, as described earlier by Lorand et al. (1972) was used to assay for TG activity and monitor the effects of the inhibitors. Unit of transglutaminase activity is 1nmol of putrescine incorporated per hour.

### Collagen

Commercial calf skin collagen type I (Sigma-Aldrich, Poole, UK. Cat no. C9791) was used during this investigation. Native collagen samples were solubilised in 0.2M acetic acid (Fisher Scientific, Loughborough, UK. Cat no. A/0400/PB17) at 4°C with constant stirring for 24 hours before use. Neutralisation of the collagen mixture was performed using a [8:1:1] ratio of [collagen: 10X DMEM: 0.2M HEPES buffer] respectively to a final of pH 7.2. Tissue culture plastic was then covered using this collagen mix (recommended at 6-10µg/cm²) before being placed into a humidified-atmosphere incubator for 12 hours to allow gelation to occur. In general, 50µl of the collagen mix was added to each well of a 96 well plate. Plates were used within 48 hours of the collagen matrix formation.

### Modified Collagen by Transglutaminase

Neutralised collagen mixture was subjected to treatment by both tissue and microbial TG. Samples of the neutralised collagen were treated with 50-1000µg/ml of tTG, in a reaction mix consisting of 2mM DTT and 5mM CaCl₂ in 10mM Tris buffer (pH 7.4). The reaction mixture for the microbial enzyme simply consisted of 10mM Tris buffer (pH 7.4). Incorporated fibronectin (Sigma-Aldrich, Poole, UK. Cat no. F0895) was used at concentrations of 5µg/ml and 50µg/ml. Transglutaminase was always added last to the collagen-reaction mix to minimise any self-imposed cross-linking. Controls using 10mM EDTA (to block tTG activity) and an active site-directed inhibitor 1,3-dimethyl-2-(2-oxopropylsulfanyl)-3H-1,3-diazol-1-ium-chloride ('R283', Nottingham Trent University, UK) were also included in each assay. For 96 well plates, 50µl of the pre-treated collagen mixture was added to each well before being placed into a humidified-atmosphere incubator, at 37°C and with 5% CO2, for 8 hours. On removal, wells were washed twice with sterile distilled water and used immediately.

### Determination of ε-(γ-glutamyl)lysine Cross-link

Cross-linked and native samples of collagen were proteolytically digested by a modification of the methods of Griffin and Wilson (1984), which included an initial digestion with microbial collagenase (Clostridium histolyticum; 1mg/ml, Sigma-Aldrich, Poole, UK. Cat no. C9891), prior to the addition of further proteases. After digestion, samples were freeze-dried and then resuspended in 0.1M HCl and sonicated for 2min to aid dispersion. An aliquot (10-90ml) was mixed with loading buffer (0.2M lithium citrate, 0.1 % phenol pH 2.2) and loaded onto a Dionex DC-4A resin column 0.5cm x 20cm using a Pharmacia Alpha Plus amino acid analyser. The buffer elution profile was as shown in the table below. Derivatisation was performed post column using o-pthaldialdehyde (0.8M boric acid, 0.78M potassium hydroxide, 600 mg/ml o-phthaldialdehyde, 0.5% (v/v) methanol, 0.75% (v/v) 2-mercaptoethanol, 0.35% (v/v) Brij 30) and the absorbance was measured at 450nm. Dipeptide was determined by addition of known amounts of ε(γ-glutamyl)lysine to the sample and comparing peak areas.

| *Time (min)* | *Buffer* | *Column temperature* |
|---|---|---|
| 0-9 | 1 | 25°C |
| 9-32 | 2 | 25°C |
| 32-67 | 3 | 25°C |
| 67-107 | 3 | 25°C |
| 107-123 | 6 | 75°C |
| 123-135 | 1 | 75°C |
| 135-147 | 1 | 65°C |
| 147-159 | 1 | 35°C |
| 159-171 | 1 | 25°C |

| | | |
|---|---|---|
| Buffer 1: 0.2M lithium citrate, 0.1% phenol, 1.5% (v/v) propan-2-ol pH 2.8. Buffer 2: 0.3M lithium citrate, 0.1% phenol, 1.5% (v/v) propan-2-ol pH 3.0. Buffer 3: 0.6M lithium citrate, 0.1% phenol pH 3.0. Buffer 6: 0.3M lithium hydroxide. | | |

### Coomassie Blue Staining Assay for Cell Culture

Native and pre-treated collagen samples gels were plated out at 50 µl per well of a 96-well plate. 100µl of a 2 x10⁴ cells/ml cell homogenate, cultured in complete media, were added to the wells in triplicates. Plates were then kept in a humidified-atmosphere incubator for the relevant time point(s). After incubation, cells were removed from the collagen matrix by addition of 0.5% (w/v) Na-deoxycholate in 10mM Tris-HCl. A rinse with distilled water was performed before the collagen samples were stained with a 0.1% (w/v) Coomassie Brilliant blue stain solution (50% (v/v) methanol; 10% (v/v) acetic acid; 40% (v/v) dH₂O). Samples were allowed to stain for 5 minutes before a further rinse with distilled water. Unstained areas, which appeared lighter blue, give an indication of collagen degradation by cells. Two separate fixed-size random fields per triplicate samples were photographed using an Olympus microscope and digital camera.

### Protein Concentration

The total protein contents of the collagen samples were determined by the Lowry method (Lowry et al., 1951) using the Bio-Rad DC protein assay kit (Bio-Rad Laboratories, Hertfordshire, UK. Cat no. 500-0120). When using buffers containing a high percentage of SDS or other detergents, the Bicinchoninic acid assay kit (Sigma-Aldrich, Poole, UK. Cat no. BCA-1) was used (Brown et al., 1989).

### Collagenase Degradation of Matrices

Collagen substrates were subjected to digestive treatment with both a 100µl of a 1mg/ml microbial collagenase solution (Clostridium histolyticum, Sigma-Aldrich, Poole, UK. Cat no. C9891) followed by 100µl 0.25% (w/v) trypsin/2mM EDTA solution in PBS solution for 24 hours at 37°C. Samples were washed twice with PBS followed by a wash with distilled water before the enzymatic digestion treatment.

### Zymosraphy

Gelatin and collagen zymography were carried out according to the following method, adapted from Herron et al, 1986. Resolving gels were mixed with the following components, in order: 1ml of 5mgml-1 Type I collagen solution (Sigma C9791) in 20mM acetic acid (for collagen zymography)/1ml of 5mgml-1 porcine gelatin (Sigma G2625) in H₂O (for gelatin zymography), 3.1ml H₂O, 2.5ml of 1.5M Tris HCl pH 8.8, 3.33ml of 29% acrylamide/1% N,N'-methylene bisacrylamide, 50µl of 10% ammonium persulphate, 10µl of N,N,N',N'-tetramethylethylenediamine (TEMED). SDS was found to cause precipitation of the collagen and so was not added to the resolving gel. Stacking gels were poured in the usual way ie. 0.65ml of 29% acrylamide/1% N,N'-methylene bisacrylamide, 3ml H₂O, 1.25ml 0.5M TrisHCl pH 6.8, 50µl of 10% SDS, 25µl of 10% ammonium persulphate, 5µl of TEMED.

Samples containing MMPs were diluted 1:1 with loading buffer (1M TrisHCl pH 6.8, 50% glycerol, 0.4% bromophenol blue) and electrophoresed at 100V in standard Laemmli running buffer (24mM TrisHCl, 192mM glycine, 3.47mM SDS, pH 8.3), avoiding overheating (approx 4-5h). After electrophoresis, gels were washed twice, with shaking, for 30min each in 200ml of 2.5% Triton X-100, to remove SDS and recover MMP activity. The gels were then placed in digestion buffer (100mM TrisHCl, 5mM CaCl₂, 0.005% Brij-35, 1µM ZnCl₂, 0.001% NaN₃, pH 8) for 16-48h at 37°C. Gels were stained with 0.2% Coomassie brilliant blue R-250 in 50% ethanol, 10% acetic acid for 2h and destained by microwaving for 15min (full power 850W) in 3 changes of deionised H2O.

### Determination of collagen fibril formation rate

Collagen fibrillogenesis was monitored by measuring the absorbance (turbidity) at 325nm using a PYE Unicam SP1800 UV spectrophotometer.

### Statistical Analysis of Data

Differences between datasets (shown as mean ±S.D.) were determined by the Student's t-test at a significance level of p< 0.05.

### Results

### Cross-linking of Collagen by Microbial and Tissue Transglutaminases

Native collagen (type I) was treated with tTG and mTG to catalyse the formation of ε-(γ-glutamyl)lysine cross-linking. Table 1 documents the results from the ion exchange analyses of the native and TG-treated collagen, giving the extent of cross-linking for each of the TG treatments. Treatment of collagen with increasing concentrations of TG leads to a corresponding increase of the amount of ε-(γ-glutamyl)lysine bonds present- with up to 1mol of cross-link per mol of collagen monomer. Treatment with mTG, gave a much greater increase (almost two-fold) of the amount of isopeptide formed for the equivalent (µg of protein) TG concentration used. It can also be seen that on incorporating fibronectin into the collagen via TG, an increase in isopeptide bonds occurs with the corresponding increase of fibronectin concentration. However, interestingly, there appears to be a decrease in the total amount of isopeptide formed for the fibronectin variants as compared to the equivalent collagen-TG only samples.

**Table 1. Transglutaminase mediated cross-linking of collagen type I and incorporation of fibronectin. Collagen samples were initially prepared at 6mg/ml. Both tTG and mTG were used at concentrations of 50-1000µg/ml (activities: tTG: 11500-13000 Units/mg; mTG: 16000-25000 Units/mg). Fibronectin was incorporated at 5µg/ml and 50µg/ml. The cross-linking reaction was allowed to proceed in a humidified-atmosphere incubator overnight at 37°C and with 5% CO2.**

| | | **nmol of cross-link/± relative change** | | |
|---|---|---|---|---|
| **Sample** | **TG conc. (µg/ml)^{s}** | **mg protein sample** | **to native collagen*** | **mol cross-link/mol of collagen⁺** |
| Collagen | - | 0.16 | - | 0.02 |
| Coll-tTG | 50 | 1.09 | 6.81 | 0.13 |
| Coll-tTG | 100 | 2.40 | 15.00 | 0.29 |
| Coll-tTG | 200 | 4.60 | 28.75 | 0.55 |
| Coll-tTG | 500 | 5.40 | 33.75 | 0.65 |
| Coll-tTG | 1000 | 8.90 | 55.63 | 1.07 |
| Coll-mTG | 10 | 0.90 | 5.63 | 0.11 |
| Coll-mTG | 50 | 2.00 | 12.5 | 0.24 |
| Coll-mTG | 200 | 4.90 | 30.63 | 0.59 |
| Coll-mTG | 500 | 8.40 | 52.50 | 1.00 |
| Coll-tTG-Fn (5µg/ml) | 100 | 0.49 | 3.06 | 0.06 |
| Coll-tTG-Fn (50µg/ml) | 100 | 1.02 | 6.38 | 0.12 |
| Coll-mTG-Fn (5µg/ml) | 100 | 0.74 | 4.63 | 0.09 |
| Coll-mTG-Fn (50µg/ml) | 100 | 0.78 | 4.88 | 0.09 |

| | | | | |
|---|---|---|---|---|
| + Mw collagen: 120kD * native collagen = 0.16nmol crosslink $ TG activity: tTG = 11500-13000 Units/mg; mTG =16000-25000 Units/mg | | | | |

### Effect of transglutaminases on collagen fibril formation

To determine the effect of transglutaminase on collagen fibrillogenesis, fibril formation after neutralisation was monitored by measuring absorbance at 325nm, as a measure of turbidity. In the case of collagen types I and III (see Figures 1 and 2, respectively), addition of either 50µg or 250µg of mTG or tTG resulted in a significant reduction in the time taken to achieve gel formation. However, type I collagen gels reached a lower final level of turbidity after treatment with transglutaminases compared to untreated gels, whereas type III collagen gels reached a higher final level of turbidity after treatment with transglutaminases compared to untreated gels. Inhibition of transglutaminase activity with the active site directed inhibitor *N-*Benzyloxycarbonyl-*L-*phenylalanyl-6-dimethylsulfonium-5-oxo-*L-*nor-leucine bromide salt ('R281', a synthetic CBZ-glutaminyl glycine analogue) abolished these effects.

### Resistance of Native and Cross-linked Collagen to HOB Cell Mediated Degradation

The capacity of HOB cells to degrade collagen, via endogenous proteases was assessed. Figure 3 presents a selection of digital photographs of the native and TG-treated collagen gels, when cultured with HOB cells for up to a 72-hour period, and the collagen then stained with Coomassie blue after removal of cells. Degradation of collagen occurs just 24 hours after the HOB cells were seeded onto the collagen. In contrast, with both the tTG and mTG-pre-treated collagen samples, degradation is at a much slower rate, with a higher amount of residual collagen remaining as judged by the amount of Coomassie blue staining. Hence, collagen treated with 50µg/ml TG (activities: tTG: 11500 Units/mg; mTG: 16000 Units/mg) showed a greater resistance to cell mediated degradation as compared to the native collagen. Comparison of the residual blue staining suggests that the mTG treated collagen shows slightly more resilience to HOB-cell degradation than the tTG-treated variant. When residual protein concentration remaining was assessed following proteolytic digestion, this confirmed the significant increased resistance of the TG cross-linked collagen to cell mediated degradation (p< 0.05). However, very little differences can be seen between the collagen cross-linked by the different transglutaminases (Figure 4).

### Resistance of Native and Cross-linked Collagen to HFDF Cell Mediated Degradation

The capacity of HFDF cells to degrade collagen, via endogenous proteases was also assessed. Figure 5 presents a selection of digital photographs of the native and TG-treated collagen gels, when cultured with HFDF cells for up to a 72-hour period. The collagen was then stained with Coomassie blue after removal of the cells. Degradation of the collagen occurs just 24 hours after the HFDF cells were seeded onto the collagen- with almost negligible residual gel remaining after 72 hours- much greater activity than the HOB cells. In contrast, with both the tTG and mTG-pre-treated collagen samples, degradation is a much slower rate and with a much higher amount of residual collagen remaining as judged by the amount of Coomassie blue staining. Hence, collagen treated with 50µg/ml TG (activities: tTG: 11500 Units/mg; mTG: 16000 Units/mg) showed a much greater resistance to HFDF cell mediated degradation as compared to the native collagen. As found with HOB cells, when residual protein concentration remaining was assessed following proteolytic digestion, this confirmed the increased resistance of cross-linked collagen to cell mediated degradation. However, in this case, a significant difference (p< 0.05) can be seen between the collagen cross-linked by the different TGs (Figure 6); it appears that mTG treated collagen shows a slightly more resilience to cell mediated degradation than the tTG-treated variant.

### Matrix metalloproteinases secreted by HFDF cells grown on transglutaminase collagen matrices

Following growth on type I collagen, fibroblasts showed an induction of a wide array of collagenases and gelatinases when compared with growth on tissue culture plasticware alone (figure 7). After growth on transglutaminase crosslinked type I collagen, the induction of active MMP1 (45kDa) is much less pronounced compared to growth on native collagen, whereas the induction of active MMP2 (66kDa) and MMP9 (86kDa) was increased. Transglutaminase crosslinking appeared to alter the MMP expression profile in a manner consistent with an increase in gelatin character. It is probable that transglutaminase crosslinked collagen matrix is interpreted in a different manner by the fibroblasts and leads to an alternative cellular response, probably explaining its resistance to cellular degradation.

### Resistance of Cross-linked and Fibronectin-Incorporated Collage to HOB and HFDF Cell Mediated Degradation

The capacity of HOB and HFDF cells to degrade the TG-treated and fibronectin incorporated collagen, via endogenous proteases was also assessed. On removal of the cells, after a 72-hour culture period, and staining with Coomassie blue, it can be seen that differences exist on comparing the TG-cross-linked collagen and the fibronectin-TG-incorporated collagen (100µg/ml of TG at activities of: activities: tTG: 11500 Units/mg; mTG: 16000 Units/mg). Figure 8 presents the residual protein concentration of the samples following the cell mediated proteolytic digestion. It can be seen that significant differences exist for both tTG-Fn and mTG-Fn treated matrices compared to the normal TG-treated collagen (p< 0.05) after 72 hours of culture. Interestingly, however, there appears to be no considerable difference in the resistance of the substrate when fibronectin concentration is increased.

### Proliferation Rates of HOB and HFDF Cells on Native, TG-treated and TG-FN Incorporated Collagen Substrates

The number of viable HOB and HFDF cells on native, TG-treated and TG-FN incorporated collagen matrices (50-100µg/ml of TG; activities: tTG: 11500 Units/mg; mTG: 16000 Units/mg) were monitored using the CellTiter reagent assay kit according to the manufacturer's instructions. It can be seen from Figure 9A that proliferation for the HOB cells is enhanced on the 50µg/ml TG-treated collagen substrates- both variants showing a higher cell density over 72 hours than that found with native collagen. For long term survival, the HOB cells also remained more viable after 168 hours of culture on the TG-treated collagens. In comparison, the HFDF cells (Figure 9B) demonstrated a significantly greater increase (p<0.05) in cell number on the TG-treated collagens, especially during the initial 72 hours of culture. However, for long term survival there is very little difference between the different collagens as the cells reach confluency with greater loss of cell viability in the tTG cross-linked collagen.

It can be seen from Figure 9C that proliferation for the HOB cells is also enhanced on the 100µg/ml TG-treated collagen substrates- both variants showing a higher cell density than that of native collagen- with the tissue transglutaminase variant providing the optimum after 60 hours. In both cases, enhancement of the long term culture viability is experienced with the TG-treated collagens. In comparison, the HFDF cells (Figure 9D) demonstrated a considerable difference during the initial 48 hours of culture (p< 0.05). The TG-treated collagen substrates allow a greater rate of cell viability to be achieved throughout the 196-hour culture period; increasing cell density rates by 15%. Microbial-treated collagen shows a slight advantage compared to the tissue-TG treated collagen. In general, significant improvements are observed when the transglutaminase concentration is increased.

The number of viable HOB and HFDF cells on cross-linked collagen substrates incorporated with fibronectin (5µg/ml and 50µg/ml) can be seen from Figure 9E and 9F respectively. In both cases, FN-incorporated matrices show a significant improvement (p< 0.05) in the cell density during the early hours of culture (24hours). However, interestingly, collagen substrates treated with 50µg/ml of FN appears to make no significant difference (p< 0.05) to the cell viability of both HOB and HFDF cells throughout culture.

### Attachment Characteristics of HOB and HFDF Cells on Native, TG-treated and TG-FN Incorporated Collagen Substrates

Figures 10A to 10F show the short-term cell-attachment characteristics of HOB and HFDF cells when cultured on native, TG-treated and TG-FN incorporated collagen substrates (10A and 10B correspond to 50µg/ml of TG; 10C to 10F corresponds to 100µg/ml of TG; activities: tTG: 11500 Units/mg; mTG: 16000 Units/mg) as monitored using light microscopy followed by May-Grunwald/Giemsa staining. Increased numbers of cells can be seen to be attached when cultured on transglutaminase cross-linked collagen. For the HOB cells, comparable cell attachment characteristics can be seen on both 50 and 100µg/ml TG-treated collagens (Figure 10A and 10C) giving a significant increase of about ~20% in attached cells for the corresponding time points over the non-crosslinked collagen (p< 0.05). Comparable enhancement in cell attachment on the cross-linked collagens were also observed for the HFDF cells (p< 0.05) (Figure 10B and 10D). In general, matrices incorporated with fibronectin show a slight enhancement in the attachment characteristics for both HOB and HFDF cells (p < 0.05) during short-term culture- with the exception of matrices treated with 50µg/ml FN; these showing no significant changes (p < 0.05) (Figures 10E and 10F).

### Spreading Characteristics of HOB and HFDF Cells on Native, TG-treated and TG-FN Incorporated Collagen Substrates

Figures 11A to 11F show the short-term cell-spreading characteristics of HOB and HFDF cells when cultured on native, TG-treated and TG-FN incorporated collagen substrates (11A and 11B correspond to 50µg/ml of TG; 11C to 11F corresponds to 100µg/ml of TG; activities: tTG: 11500 Units/mg; mTG: 16000 Units/mg) as monitored using light microscopy followed by May-Grunwald/Giemsa staining (Figure 12). Increased numbers of cells can be seen to be spread when cultured on 50µg/ml transglutaminase cross-linked collagen. In the case of the HOB cells, a comparable increase of ~5% in the spreading of the HOB cells, at each time point, can be seen on both of the TG-treated collagens (Figure 11A). In contrast, the HFDF cells show much more distinct and significant spreading characteristics on the 50µg/ml TG-treated collagen- increases of at least 10% can be noted for both of the TG-treated variants (Figure 11B) (p< 0.05).

A further increase in the number of spread cells can be identified on 100µg/ml transglutaminase cross-linked collagen. In the case of HOB cells, a comparable difference of ~5% increase ion spread cells can be noted (Figure 11C)- this behaviour increases with time for extended culture. In contrast for the HFDF cells, although there is still an increase in the spreading characteristics on the TG-treated collagen, a much more distinct and significant behaviour can be identified on the tissue enzyme treated collagen; spreading characteristics increase by 15% for many of the time points. Contrastingly, the microbial-treated collagen shows only a slight improvement in the spreading characteristics (Figure 11D) (p< 0.05).

In the case of TG-FN incorporated matrices, it can be seen that a significant enhancement of the spreading characteristic is noted on 5µg/ml FN substrates for HOB and HFDF cells (p < 0.05) (Figures 11E and 11F respectively). However, for both cases of TG-FN (50µg/ml), a decrease in the spreading characteristics is noted when compared to the normal TG-cross-linked substrate.

### Alkaline Phosphatase Activity of HOB Cells Cultured on Native and TG-treated Collagen

Figure 13 shows ALP activity of HOB cells cultured on native and TG-treated collagen (50-250µg/ml of tTG and mTG; activities: tTG: 11500 Units/mg; mTG: 16000 Units/mg). Increases in ALP activity were observed in all the TG-crosslinked collagens- the greatest increase seen with the collagen-tTG substrate followed by the collagen-mTG. Typically, an increase in the concentration of TG improved the ALP activity of the HOB cells (p< 0.05). Interestingly however, the collagen treated with the higher concentration of mTG (250µg/ml) appears to reduce the corresponding amount of ALP activity when compared to tTG.

### Summary & Conclusions

The above results demonstrate the following:
- Both microbial and tissue transglutaminases are able to crosslink type I collagen.
- Crosslinking of collagen results in an improvement in the resistance to degradation by different cell types.
- Cells show improved attachment, spreading and proliferation when cultured on collagen treated with either microbial or tissue transglutaminases; this effect is enhanced when fibronectin is also crosslinked to the collagen.
- Treatment of type I and type III collagens with either microbial or tissue transglutaminases immediately after neutralisation from acidic solution, causes an increase in gelation/fibrillogenesis rate.

These data, taken together, show that transglutaminase treated collagen or collagen/fibronectin matrices offer a significant advantage over standard collagen as biomaterials for *in vivo* use with regard to both biological and physical stability, and biocompatibility.

Collagen, with its superior biocompatibility compared to other natural polymers, and its excellent safety due to its biological characteristics, such as biodegradability and weak antigenicity, has made collagen the primary resource in medical applications (Lee et al., 2001). Collagen isolated from rat tail tendon or foetal calf skin has frequently been used successfully as a support and adhesion substance in many tissue culture systems for many types of cell lines including osteoblasts (Schuman et al., 1994; Lynch et al., 1995) and fibroblasts (Ivarsson et al., 1998). Additionally, Mizuno et al. (1997) have also reported that type I collagen matrices offer a favourable environment for the induction of osteoblastic differentiation in vitro. However, the use of natural polymers as potential biomaterials, matrices or scaffolds for cell based applications in tissue engineering is often restricted by its poor mechanical characteristics and loss of biological properties during formulation (Hubbell, 1995). The major deciding factor, and primary disadvantage, of many biocomposites concerns the requirement for chemical cross-linking of the constituent monomers to increase stability and physical performance during manufacture, thus raising concerns about the issues of toxicity due to residual catalysts, initiators and unreacted or partially reacted cross-lining agents in the final polymer (Coombes et al., 2001). Collagen, like many natural polymers once extracted from its original source and then reprocessed, suffers from weak mechanical properties, thermal instability and ease of proteolytic breakdown. However, it has been demonstrated that transglutaminases are able to crosslink native collagen type I by catalysing the formation of isopeptide bonds.

Here, it is demonstrated that TG-modified collagen demonstrates greater resistance to cell secreted proteases and, as a consequence, improved resistance to cell mediated degradation from cultured HOB and HFDF cells. Crosslinking of the collagen alters the MMP expression profile of HFDF cells grown on these modified substrates, with a reduction of active MMP1 and a corresponding increase in active MMP2 when compared to growth on unmodified collagen. This is probably due to altered signalling of the nature of the extracellular matrix caused by transglutaminase modification, with cells recognising it less as fibrillar collagen. Indeed, transglutaminase treatment of type I collagen results in a gel that does not achieve as high a turbidity as untreated collagen, possibly indicating a reduction in fibrillar form. In contrast, type III collagen shows an increased turbidity with transglutaminase treatment.

It has also been demonstrated that the modified collagen is more biocompatible to a wide variety of cells, as shown using HOB and HFDF cells. Not only does it enhance the proliferation rates of the cells, but cell attachment and cell spreading of these cells is also increased when compared to native collagen gels. Additionally, long-term growth and survival are maintained with respect to applications in bone repair. Importantly, HOB cells are able to differentiate more quickly on TG-modified collagens as demonstrated by the corresponding increases in ALP activities. Furthermore, on incorporating fibronectin into the collagen substrates, further enhancement of cell properties of proliferation, spreading and attachment are experienced.

In conclusion, transglutaminase-mediated cross-linking of collagen has the potential to improve the physical and mechanical properties of native collagen by the formation of stabilising cross-links. Importantly, however, TG increases the resistance of the collagen to cell degradation and, in addition, enhances the biocompatibility of the substrate by facilitating increased cell enhancing proliferation and also allowing greater attachment and spreading of cells.

### REFERENCES

Aeschlimann, D. and Paulsson, M. (1994): Transglutaminases: Protein Cross-Linking Enzymes in Tissues and Body Fluids. Thrombosis and Haemostasis, 71(4), 402-425.
Barbani N, Giusti P, Lazzeri L, Polacco G & Pizzirani G (1995). Bioartificial materials based on collagen: 1. Collagen cross-linking with gaseous glutaraldehyde. Journal of Biomaterials Science-Polymer Edition 7 (6): 461-469
Bell E, Ehrlich P, Buttle DJ & Nakatsuji T (1981). Living tissue formed in vitro and accepted as skin-equivalent of full thickness. Science 221: 1052
Ben-Slimane S, Guidoin R, Marceau D, Merhi Y, King MW, Sigot-Luizard MF. 1988. J Eur. Surj. Res. 20:18-28
Bradley WG & Wilkes GL (1977). Some mechanical property considerations of reconstituted collagen for drug release supports. Biomaterials Medical Devices and Artificial Organs 5: 159-175
Burke JF, Yannas IV, Quimby WC, Bondoc CC & Jung WK (1981). Successful use of a physiologically acceptable artificial skin in the treatment of extensive burn injury. Annals of Surgery 194: 413-448
Chvapil M, Speer DP, Holubec H, Chvapil TA, King DH (1993). Collagen-fibers as a temporary scaffold for replacement of ACL in goats. Journal of Biomedical Materials Research 27 (3): 313-325
Collighan R, Cortez J & Griffin M (2002). The biotechnological applications of transglutaminases. Minerva Biotecnologica 14 (2): 143-148
Coombes AGA, Verderio E, Shaw B, Li X, Griffin M, Downes S. (2001). Biocomposites of non-crosslinked natural and synthetic polymers. Biomaterials 23: 2113-2118
Dunn MW, Stenzel KH, Rubin AL, & Miyata T (1969). Collagen implants in the vitreous. Archives of Ophthamology 82: 840-844
Einerson NJ, Stevens KR & Kao WYJ (2003). Synthesis and physicochemical analysis of gelatin-based hydrogels for drug carrier matrices. Biomaterials 24 (3): 509-523
Gentile et al., (1991) Isolation and characterization of cDNA clones to mouse macrophage and human endothelial cell tissue transglutaminases. J. Biol. Chem. 266(1) 478-483
Goo HC, Hwang YS, Choi YR, Cho HN & Suh H (2003). Development of collagenase-resistant collagen and its interaction with adult human dermal fibroblasts. Biomaterials 24 (28): 5099-5113
Gorham SD, LightND, Diamond AM, Willins MJ, Bailey AJ, Wess TJ & Leslie NJ (1992). Effect of chemical modifications on the susceptibility of collagen to proteolysis: 2. Dehydrothermal cross-linking. International Journal of Biological Macromolecules 14 (3): 129-138
Greenberg, C.S., Birckbichler, P.J. and Rice, R.H. (1991): Transglutaminase: Multifunctional Crosslinking Enzymes that Stabilise Tissues. FASEB, 5, 3071-3077
Griffin M, Wilson J (1984). Detection of ε-(γ-glutamyl)lysine. Molecular and Cellular Biochemistry 58: 37-49
Grundmann et al. (1986) Characterization of cDNA coding for human factor XIIIa. Proc. Natl. Acad. Sci. USA 83(21), 8024-8028
Harkness RD (1966). Collagen. Scientific Progress (Oxford) 54: 257-274
Hubbel JA (1995). Biomaterials in tissue engineering. Biotechnology 13: 565-576
Ito A, Mase A, Takizawa Y, Shinkai M, Honda H, Hata K, Ueda M & Kobayashi T (2003). Transglutaminase-mediated gelatin matrices incorporating cell adhesion factors as a biomaterial for tissue engineering. Journal of Bioscience and Bioengineering 95 (2): 196-199
Ivarsson M, McWhirter A, Borg TK, Rubin K. (1998). Type I collagen synthesis in cultured human fibroblasts: regulation by cell spreading, platelet-derived growth factor and interactions with collagen fibers. Matrix Biology 16: 409-425
Johnson TS, Skill NJ, EI Nahas AM, Oldroyd SD, Thomas GL, Douthwaite JA, Haylor JL, Griffin M (1999). Transglutaminase transcription and antigen translocation in experimental renal scarring. J. Am. Soc. Nephrol., 10: 2146-2157
Jones RA, Nicholas B, Mian S, Davies PJA, Griffin M (1997). Reduced expression of tissue transglutaminase in a human endothelial cell line leads to changes in cell spreading, cell adhesion and reduced polymerisation of fibronectin. J. Cell Sci 110: 2461-2472
Lee CH, Singla A & Lee Y (2001). Biomedical applications of collagen. International Journal of Pharmaceutics 221 (1-2): 1-22
Lorand et al. (1972) A filter paper assay for transamidating enzymes using radioactive amine substrates. Analytical Biochemistry 50,623
Lowry OH, Rosebrough NJ, Farr AL, Randall RJ (1951). Journal of Biological Chemistry 193: 265-275
Lynch MP, Stein JL, Stein GS, Lian JB. (1995). The influence of type I collagen on the development and maintenance of the osteoblast phenotype in primary and passaged rat calvarial osteoblasts: modification of expression of genes supporting cell growth, adhesion and extracellular matrix mineralization. Experimental Cell Research 216: 35-45
Matsuda S, Iwata H, Se N & Ikada Y (1999). Bioadhesion of gelatin films crosslinked with glutaraldehyde. Journal of Biomedical Materials Research 45 (1): 20-27
Miyata T, Sohde T, Ruben AL & Stenzel KH (1971). Effects of ultraviolet radiation on native and telopeptide-poor collagen. biochimica et biophysica acta 229: 672-280
Mizuno M, Shindo M, Kobayashi D, Tsuruga E, Amemiya A, Kuboki Y (1997). Osteogenesis by bone marrow stromal cells maintained on type I collagen matrix gels in vivo. Bone 20(2): 101-107
Nimni ME, Cheung D, Strates B, Kodama M & Sheikh K (1988). Bioprosthesis derived from crosslinked and chemically modified collagenous tissue. In: Nimni, ME (Ed.), Collagen Biotechnology, vol. III. CRC Press, Boca Raton, FL, p 1-38
Petite H, Rault I, Hue A, Menasche P & Herbage D (1990). Use of the acyl azide method for cross-linking collagen-rich tissues such as pericardium. Journal of Biomedical Materials Research 24 (2): 179-187
Rhee SH, Hwang MH, Si HJ & Choi JY (2003). Biological activities of osteoblasts on poly(methyl methacrylate)/silica hybrid containing calcium salt. Biomaterials 24 (6): 901-906
Richardson et al., 1995, Methods in Molecular Biology Vol 39, J Walker ed., Humana Press, Totowa, NJ
Rudennan RJ, Wade CWR, Shepard WD & Leonard F (1973). Prolonged resorption of collagen sponges: vapor-phase treatment with formaldehyde. Journal of Biomedical Materials Research 7: 263-265
Sachlos E, Reis N, Ainsley C, Derby B & Czernuszka JT (2003). Novel collagen scaffolds with predefined internal morphology made by solid freeform fabrication. Biomaterials 24 (8): 1487-1497
Saiki et al (1988) Primer-directed enzymatic amplification of DNA with a thennostable DNA polymerase. Science 239, 487-491
Sambrook & Russell, 2001, Molecular Cloning, A Laboratory Mannual, Third Edition, Cold Spring Harbor, New York
Schmedlen KH, Masters KS & West JL (2002). Photocrosslinkable polyvinyl alcohol hydrogels that can be modified with cell adhesion peptides for use in tissue engineering. Biomaterials 23 (22): 4325-4332
Stenzel KH, Dunn MW, Ruben AL & Miyata T (1969). Collagen gels: design for vitreous replacement. Science 164: 1282-1283
Schuman L, Buma P, Verseyen D, deMan B, van der Kraan PM, van der Berg WB, Homminga GN (1995). Chondrocyte behaviour within different types of collagen gel in vitro. Biomaterials 16(10): 809-814
Tu R, Lu CL, Thyagarajan K, Wang E, Nguyen H, Shen S, Hata C & Quijano RC (1993). Kinetic-study of collagen fixation with polyepoxy fixatives. Journal of Biomedical Materials Research 27 (1): 3-9

## Claims

1. Use of a transglutaminase for improving the biocompatibility of a biomaterial comprising collagen by crosslinking the collagen-,
wherein improving the biocompatibility of the biomaterial comprises enhancing its ability to support cell attachment, cell spreading, cell proliferation and differentiation compared to non-crosslinked collagen.

2. The use according to Claim 1 wherein improving the biocompatibility of the biomaterial comprises enhancing its ability to support attachment, spreading, proliferation and/or differentiation of osteoblasts compared to non-crosslinked collagen.

3. The use according to any one of the preceding claims wherein improving the biocompatibility of the biomaterial comprises enhancing its resistance to cell-mediated degradation compared to non-crosslinked collagen.

4. The use according to Claim 34- wherein improving the biocompatibility of the biomaterial comprises enhancing its resistance to one or more protease enzymes produced by mammalian cells.

5. The use according to any one of the preceding claims wherein the biomaterial consists of substantially pure collagen.

6. The use according to any one of the preceding claims wherein the biomaterial comprises a cell adhesion factor.

7. The use according to Claim 6 wherein the cell adhesion factor is selected from the group consisting of fibronectin, fibrin, fibrillin, glycosoaminoglycans, hyaluronic acid laminin, vitronectin and elastin.

8. The use according to Claim 6 or 7 wherein the cell adhesion factor is fibronectin.

9. The use according to any one of the preceding claims wherein the biocompatible biomaterial comprises one or more additives.

10. The use according to Claim 9 wherein the additive is selected from the group consisting of polylactic acid, polyhydroxybutyrate, poly(ε-caprolactone), polyglycolic acid, polysaccharides, chitosans and silicates.

11. The use according to Claim 9 wherein the additive is selected from the group consisting of metals, bioceramics, glass, silk and biostable polymers.

12. The use according to Claim 11 wherein the biostable polymer is selected from the group consisting of polypropylene, polyurethane, polytetrafluoroethylene, poly(vinyl chloride), polyamides, poly(methylmethacrylate), polyacetal, polycarbonate, poly(-ethylene terphthalate), polyetheretherketone, and polysulfone.

13. The use according to any one of the preceding claims wherein the transglutaminase is a tissue transglutaminase.

14. The use according to any one of the preceding claims wherein the transglutaminase is a plasma transglutaminase.

15. The use according to any one of the preceding claims wherein the transglutaminase is prepared from mammalian tissue or cells.

16. The use according to Claim 15 wherein the transglutaminase is guinea pig liver tissue transglutaminase.

17. The use according to Claim 15 wherein the transglutaminase is prepared from human tissue or cells.

18. The use according to Claim 17 wherein the human tissue or cells are selected from the group consisting of lung, liver, spleen, kidney, heart muscle, skeletal muscle, eye lens, endothelial cells, erythrocytes, smooth muscle cells, bone and macrophages.

19. The use according to any one of Claims 1 to 12 wherein the transglutaminase is a microbial transglutaminase.

20. The use according to Claim 19 wherein the transglutaminase is derived or prepared from the group consisting of *Streptoverticillium mobaraenase, Streptoverticillium ladakanum, Streptoverticillium cinnamoneum, Bacillus subtilis* and *Phytophthora cactorum.*

21. The use according to any one of the preceding claims wherein the transglutaminase is a recombinant transglutaminase.

22. The use according to any one of the preceding claims wherein the transglutaminase is a variant transglutaminase.

23. The use according to any one of the preceding claims wherein the collagen is neutralised prior to treatment with the transglutaminase.

24. The use according to any one of the preceding claims wherein the transglutaminase is used at a concentration of between 50 and 1000 µg per ml of reaction mixture.

25. The use according to any one of the preceding claims wherein the collagen is present at a concentration of 3 to 6 mg/ml of reaction mixture.

26. The use according to any one of the preceding claims wherein the treatment of collagen with the transglutaminase is performed in the presence of a reducing agent.

27. The use according to any one of the preceding claims wherein the treatment of collagen with the transglutaminase is performed in the presence of calcium ions.

28. The use according to any one of the preceding claims wherein the treatment of collagen with the transglutaminase is performed in the presence of buffering agent which buffers the reaction mixture at pH 7.4.

29. The use according to any one of the preceding claims wherein treatment with the transglutaminase is performed at 37°C.

30. The use according to any one of the preceding claims wherein the biomaterial is a medical implant or wound dressing.

31. The use according to Claim 30 wherein the medical implant is artificial bone.

## Patentansprüche

1. Verwendung einer Transglutaminase zur Verbesserung der Biokompatibilität eines Kollagen enthaltenden Biomaterials durch Vernetzung des Kollagens, **dadurch gekennzeichnet, dass** die Verbesserung der Biokompatibilität des Biomaterials eine Verbesserung seiner Eigenschaft zur Unterstützung von Zellbindung, Zellwachstum, Zellvermehrung und/oder Zelldifferenzierung, verglichen mit nicht-vernetztern Kollagen, umfasst.

2. Verwendung nach Anspruch 1, **dadurch gekennzeichnet, dass** die Verbesserung der Biokompatibilität des Biomaterials eine Verbesserung seiner Eigenschaft zur Unterstützung von Bindung, Wachstum, Vermehrung und/oder Differenzierung von Osteoblasten, verglichen mit nicht-vernetztem Kollagen, umfasst.

3. Verwendung nach einem der vorhergehenden Ansprüche, **dadurch gekennzeichnet, dass** die Verbesserung der Biokompatibilität des Biomaterials eine Verbesserung seiner Resistenz gegenüber zellvermittelter Degradation, verglichen mit nicht-vernetztem Kollagen, umfasst.

4. Verwendung nach Anspruch 3, **dadurch gekennzeichnet, dass** die Verbesserung der Biokompatibilität des Biomaterials eine Verbesserung seiner Resistenz gegenüber einem oder mehreren Proteaseenzymen, die von Säugetierzellen erzeugt wurden, umfasst.

5. Verwendung nach einem der vorhergehenden Ansprüche, **dadurch gekennzeichnet, dass** das Biomaterial aus weitgehend reinem Kollagen besteht.

6. Verwendung nach einem der vorhergehenden Ansprüche, **dadurch gekennzeichnet, dass** das Biomaterial einen Zelladhäsionsfaktor umfasst.

7. Verwendung nach Anspruch 6, **dadurch gekennzeichnet, dass** der Zelladhäsionsfaktor aus einer Gruppe ausgewählt wird, welche aus Fibronektin, Fibrin, Fibrillin, Glykosaminoglykan, Hyaluronsäure, Laminin, Vitronektin und Elastin besteht.

8. Verwendung nach Anspruch 6 oder 7, **dadurch gekennzeichnet, dass** der Zelladhäsionsfaktor Fibronektin ist.

9. Verwendung nach einem der vorhergehenden Ansprüche, **dadurch gekennzeichnet, dass** das biokompatible Biomaterial eines oder mehrere Additive umfasst.

10. Verwendung nach Anspruch 9, **dadurch gekennzeichnet, dass** das Additiv aus einer Gruppe ausgewählt wird, welche aus Polylactid, Polyhydroxybuttersäure, Poly(-ε-)Caprolacton, Polyglycolidsäure, Polysacchariden, Chitosan und Silicaten besteht.

11. Verwendung nach Anspruch 9, **dadurch gekennzeichnet, dass** das Additiv aus einer Gruppe ausgewählt wird, welche aus Metallen, Biokeramiken, Glas, Seide und biostabilen Polymeren besteht.

12. Verwendung nach Anspruch 11, **dadurch gekennzeichnet, dass** das biostabile Polymer ausgewählt wird aus einer Gruppe, welche aus Polypropylen, Polyurethan, Polytetrafluorethylen, Poly(vinylchlorid), Polyamiden, Poly(methylmethacrylat), Polyoxymethylen, Polycarbonat, Poly(ethylenterephthalat), Polyetheretherketon und Polysulfon besteht.

13. Verwendung nach einem der vorhergehenden Ansprüche, **dadurch gekennzeichnet, dass** die Transglutaminase eine Gewebstransglutaminase ist.

14. Verwendung nach einem der vorhergehenden Ansprüche, **dadurch gekennzeichnet, dass** die Transglutaminase eine Plasmatransglutaminase ist.

15. Verwendung nach einem der vorhergehenden Ansprüche, **dadurch gekennzeichnet, dass** die Transglutaminase aus Säugetiergewebe oder -zellen hergestellt wird.

16. Verwendung nach Anspruch 15, **dadurch gekennzeichnet, dass** die Transglutaminase eine Meerschweincheniebergewebe-Transglutaminase ist.

17. Verwendung nach Anspruch 15, **dadurch gekennzeichnet, dass** die Transglutaminase aus menschlichem Gewebe oder menschlichen Zellen hergestellt wird.

18. Verwendung nach Anspruch 17, **dadurch gekennzeichnet, dass** das menschliche Gewebe oder die menschlichen Zellen aus der Gruppe ausgewählt werden, welche aus Lunge, Leber, Milz, Niere, Herzmuskel, Skelettmuskel, Augenlinse, Endothelzellen, Erythrozyten, glatten Muskelzellen, Knochen und Makrophagen besteht.

19. Verwendung nach einem der Ansprüche 1 bis 12, **dadurch gekennzeichnet, dass** die Transglutaminase eine mikrobielle Transglutaminase ist.

20. Verwendung nach Anspruch 19, **dadurch gekennzeichnet, dass** die Transglutaminase hergestellt oder bereitgestellt wird aus der Gruppe, welche aus *Streptoverticillium mobaraenase, Streptoverticillium ladakanum, Streptoverticillium cinnamoneum, Bacillus subtilis* und *Phytophthora cactorum* besteht.

21. Verwendung nach einem der vorhergehenden Ansprüche, **dadurch gekennzeichnet, dass** die Transglutaminase eine rekombinante Transglutaminase ist.

22. Verwendung nach einem der vorhergehenden Ansprüche, **dadurch gekennzeichnet, dass** die Transglutaminase eine Transglutaminase-Variante ist.

23. Verwendung nach einem der vorhergehenden Ansprüche, **dadurch gekennzeichnet, dass** das Kollagen vor der Behandlung mit der Transglutaminase neutralisiert wird.

24. Verwendung nach einem der vorhergehenden Ansprüche, **dadurch gekennzeichnet, dass** die Transglutaminase in einer Konzentration zwischen 50 und 1000 µg pro ml Reaktionsmischung verwendet wird.

25. Verwendung nach einem der vorhergehenden Ansprüche, **dadurch gekennzeichnet, dass** das Kollagen in einer Konzentration von 3 bis 6 mg/ml Reaktionsmischung vorhanden ist.

26. Verwendung nach einem der vorhergehenden Ansprüche, **dadurch gekennzeichnet, dass** die Behandlung von Kollagen mit der Transglutaminase unter Anwesenheit eines Reduktionsmittels durchgeführt wird.

27. Verwendung nach einem der vorhergehenden Ansprüche, **dadurch gekennzeichnet, dass** die Behandlung von Kollagen mit der Transglutaminase unter Anwesenheit von Kalziumionen durchgeführt wird.

28. Verwendung nach einem der vorhergehenden Ansprüche, **dadurch gekennzeichnet, dass** die Behandlung von Kollagen mit der Transglutaminase unter Anwesenheit eines Puffers durchgeführt wird, der die Reaktionsmischung bei pH 7.4 puffert.

29. Verwendung nach einem der vorhergehenden Ansprüche, **dadurch gekennzeichnet, dass** die Behandlung mit Transglutaminase bei 37°C durchgeführt wird.

30. Verwendung nach einem der vorhergehenden Ansprüche, **dadurch gekennzeichnet, dass** das Biomaterial ein medizinisches Implantat oder eine Wundauflage ist

31. Verwendung nach Anspruch 30, **dadurch gekennzeichnet, dass** das medizinische Implantat ein künstlicher Knochen ist.

## Revendications

1. Utilisation d'une transglutaminase pour améliorer la biocompatibilité d'un biomatériau comprenant du collagène en réticulant le collagène,
dans laquelle l'amélioration de la biocompatibilité du biomatériau comprend l'accroissement de son aptitude à supporter l'attachement cellulaire, l'étalement cellulaire, la prolifération et la différenciation cellulaires par rapport à un collagène non réticulé.

2. Utilisation selon la revendication 1, dans laquelle l'amélioration de la biocompatibilité du biomatériau comprend l'accroissement de son aptitude à supporter l'attachement, l'étalement, la prolifération et/ou la différenciation d'ostéoblastes par rapport à un collagène non réticulé.

3. Utilisation selon l'une quelconque des revendications précédentes, dans laquelle l'amélioration de la biocompatibilité du biomatériau comprend l'accroissement de sa résistance à une dégradation médiée par des cellules par rapport à un collagène non réticulé.

4. Utilisation selon la revendication 3, dans laquelle l'amélioration de la biocompatibilité du biomatériau comprend l'accroissement de sa résistance à une ou plusieurs enzymes protéases produites par des cellules de mammifères.

5. Utilisation selon l'une quelconque des revendications précédentes, dans laquelle le biomatériau consiste en collagène pratiquement pur.

6. Utilisation selon l'une quelconque des revendications précédentes, dans laquelle le biomatériau comprend un facteur d'adhésion cellulaire.

7. Utilisation selon la revendication 6, dans laquelle le facteur d'adhésion cellulaire est choisi dans le groupe constitué par la fibronectine, la fibrine, la fibrilline, les glycosoaminoglycanes, l'acide hyaluronique, la laminine, la vitronectine et l'élastine.

8. Utilisation selon la revendication 6 ou 7, dans laquelle le facteur d'adhésion cellulaire est la fibronectine.

9. Utilisation selon l'une quelconque des revendications précédentes, dans laquelle le biomatériau biocompatible comprend un ou plusieurs additifs.

10. Utilisation selon la revendication 9, dans laquelle l'additif est choisi dans le groupe constitué par un acide polylactique, un polyhydroxybutyrate, une poly(ε-caprolactone), un acide polyglycolique, les polysaccharides, les chitosanes et les silicates.

11. Utilisation selon la revendication 9, dans laquelle l'additif est choisi dans le groupe constitué par les métaux, les biocéramiques, le verre, la soie et les polymères biostables.

12. Utilisation selon la revendication 11, dans laquelle le polymère biostable est choisi dans le groupe constitué par un polypropylène, un polyuréthane, un polytétrafluoroéthylène, un poly(chlorure de vinyle), les polyamides, un poly(méthacrylate de méthyle), un polyacétal, un polycarbonate, un poly(téréphtalate d'éthylène), une polyétheréthercétone et une polysulfone.

13. Utilisation selon l'une quelconque des revendications précédentes, dans laquelle la transglutaminase est une transglutaminase tissulaire.

14. Utilisation selon l'une quelconque des revendications précédentes, dans laquelle la transglutaminase est une transglutaminase plasmatique.

15. Utilisation selon l'une quelconque des revendications précédentes, dans laquelle la transglutaminase est préparée à partir d'un tissu ou de cellules de mammifères.

16. Utilisation selon la revendication 15, dans laquelle la transglutaminase est une transglutaminase de tissu hépatique de cochon d'Inde.

17. Utilisation selon la revendication 15, dans laquelle la transglutaminase est préparée à partir d'un tissu ou de cellules humain(es).

18. Utilisation selon la revendication 17, dans laquelle le tissu ou les cellules humain(es) sont choisis dans le groupe constitué par un poumon, un foie, une rate, un rein, un muscle cardiaque, un muscle squelettique, un cristallin, des cellules endothéliales, des érythrocytes, des cellules de muscle lisse, un os et des macrophages.

19. Utilisation selon l'une quelconque des revendications 1 à 12, dans laquelle la transglutaminase est une transglutaminase microbienne.

20. Utilisation selon la revendication 19, dans laquelle la transglutaminase est dérivée ou préparée à partir du groupe constitué par *Streptoverticillium mobaraenase*, *Streptoverticillium ladakanum*, *Streptoverticillium cinnamoneum, Bacillus subtilis* et *Phytophthora cactorum*.

21. Utilisation selon l'une quelconque des revendications précédentes, dans laquelle la transglutaminase est une transglutaminase recombinante.

22. Utilisation selon l'une quelconque des revendications précédentes, dans laquelle la transglutaminase est une transglutaminase variante.

23. Utilisation selon l'une quelconque des revendications précédentes, dans laquelle le collagène est neutralisé avant le traitement avec la transglutaminase.

24. Utilisation selon l'une quelconque des revendications précédentes, dans laquelle la transglutaminase est utilisée à une concentration d'entre 50 et 1000 µg par ml de mélange réactionnel.

25. Utilisation selon l'une quelconque des revendications précédentes, dans laquelle le collagène est présent à une concentration de 3 à 6 mg/ml de mélange réactionnel.

26. Utilisation selon l'une quelconque des revendications précédentes, dans laquelle le traitement du collagène avec la transglutaminase est effectué en présence d'un agent réducteur.

27. Utilisation selon l'une quelconque des revendications précédentes, dans laquelle le traitement du collagène avec la transglutaminase est effectué en présence d'ions calcium.

28. Utilisation selon l'une quelconque des revendications précédentes, dans laquelle le traitement du collagène avec la transglutaminase est effectué en présence d'un agent tamponnant qui tamponne le mélange réactionnel à pH 7,4.

29. Utilisation selon l'une quelconque des revendications précédentes, dans laquelle le traitement avec la transglutaminase est effectué à 37 °C.

30. Utilisation selon l'une quelconque des revendications précédentes, dans laquelle le biomatériau est un implant médical ou un pansement pour blessure.

31. Utilisation selon la revendication 30, dans laquelle l'implant médical est un os artificiel.
